(19) Europäisches Patentamt
European Patent Office
Office européen des brevets

(11) Publication number: **0 105 193**
**B1**

(12) **EUROPEAN PATENT SPECIFICATION**

(45) Date of publication of patent specification: **24.08.88**

(51) Int. Cl.[4]: **C 07 C 129/12,** A 61 K 31/16

(21) Application number: **83108567.5**

(22) Date of filing: **31.08.83**

(54) **Spergualin-related compounds, processes for the preparation thereof and their use as medicaments.**

(30) Priority: **02.09.82 JP 151698/82**

(43) Date of publication of application:
**11.04.84 Bulletin 84/15**

(45) Publication of the grant of the patent:
**24.08.88 Bulletin 88/34**

(84) Designated Contracting States:
**AT BE CH DE FR GB IT LI LU NL SE**

(56) References cited:
**GB-A-2 084 999**

(73) Proprietor: **MICROBIAL CHEMISTRY RESEARCH FOUNDATION**
**14-23, Kamiosaki 3 Chome Shinagawa-ku**
**Tokyo 141 (JP)**

(72) Inventor: **Umezawa, Hamao, Prof.**
**4-23, Toyotama-kita**
**Nerima-ku Tokyo (JP)**
Inventor: **Takeuchi, Tomio**
**5-1-11, Higashi-Gotanda**
**Shinagawa-ku Tokyo (JP)**
Inventor: **Nishizawa, Rinzo**
**3-18-14, Sugamo**
**Toshima-ku Tokyo (JP)**
Inventor: **Takahashi, Katsutoshi**
**3-10-8-512, Kamiya**
**Kita-ku Tokyo (JP)**
Inventor: **Nakamura, Teruya**
**831-6, Nomura-cho**
**Kusatsu City Shiga Prefecture (JP)**
Inventor: **Umeda, Yoshihisa**
**2-27-21, Jinryo**
**Ohtsu City Shiga Prefecture (JP)**

(74) Representative: **Meyer-Dulheuer, Karl-Hermann, Dr. et al**
**HOECHST Aktiengesellschaft Zentrale**
**Patentabteilung Postfach 80 03 20**
**D-6230 Frankfurt/Main 80 (DE)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

EP 0 105 193 B1

**Description**

This invention relates to novel spergualin-related compounds, and processes for preparing these compounds. The spergualin-related compounds have the following general formula (I)

$$H_2N\underset{\underset{NH}{\|}}{C}NH(CH_2)_m\underset{\underset{R_1}{|}}{C}HCH_2CO-R_2-NH(CH_2)_4NH(CH_2)_3NH_2 \qquad (I)$$

wherein $R_1$ represents a hydrogen atom, a hydroxyl group, or an aliphatic acyloxy group with 1 to 10 carbon atoms; $R_2$ represents a residue of an α- or ω-amino acid remaining after removing one of the hydrogen atoms from the α- or ω-amino group and the hydroxyl group from the α-carboxyl group, having the formula IV

$$-NH-\overset{\overset{X}{|}}{C}H-(CH_2)_n-CO- \qquad (IV)$$

where X represents a hydrogen atom or a straight-chain or branched alkyl group having 1 to 6 carbon atoms, said alkyl group optionally having as a substituent a hydroxyl, carboxyl, lower alkoxycarbonyl, guanidino, phenyl or imidazole group and n denotes an integer of 0 to 2; or having the formula V

```
   ,X,
  /   \
-N  -  CH-CO-        (V)
```

wherein X represents an alkylene group having up to 3 carbon atoms; said $R_2$ being attached to the adjacent carbonyl group and amino group by amide bonds; and m denotes an integer of 4 to 6, and a physiologically acceptable salt thereof.

The invention also relates to processes for preparing the spergualin-related compounds of the general formula (I) and salts thereof, which comprise removing protective groups from protected spergualin-related compounds of the following formula (II)

$$H_2N\underset{\underset{NH}{\|}}{C}NH(CH_2)_m\underset{\underset{R_1}{|}}{C}HCH_2CO-R'_2-NH(CH_2)_4\overset{\overset{R_3}{|}}{N}(CH_2)_3NH-R_4 \qquad (II)$$

wherein $R_1$ is as defined; $R_2'$ has the meaning of $R_2$ with the proviso, that said α- or ω-amino acid is optionally protected, if it has a functional group as a substituent; $R_3$ and $R_4$ are identical or different and each represents a protective group for the amino group, and m is as defined, by usual methods.

Spergualin is a compound which has been isolated from the filtrate of a culture medium after cultivation of a spergualin-producing strain belonging to the genus Bacillus. Spergualin has the following structure:

$$H_2N\underset{\underset{NH}{\|}}{C}NH(CH_2)_4\underset{\underset{OH}{|}}{C}HCH_2CONH-\underset{\underset{OH}{|}}{C}HCO-NH(CH_2)_4-NH-(CH_2)_3-NH_2 \qquad (III)$$

Spergualin inhibits the growth of gram-positive and gram-negative bacteria, and shows marked efficacy and prolongs the lives of animals in the treatment of mouse leukemia L—1210, mouse leukemia FL—4, Ehrlich carcinoma and sarcoma 180 (S—180). It is thus a compound expected to serve as an antitumor agent (see Japanese-Open Patent Publication No. 48957/72).

It is also known that spergualin can be obtained by synthesis [J. Antibiotics, Vol. 34, 1625 (1981)]. 15-Deoxyspergualin deoxidized at the 15-position, is also known to have the same effects as described above.

These compounds exhibit an excellent carcinostatic action, but they have poor stability in the form of aqueous solutions, which poses a great impediment to their practical application.

It has now been found that spergualin-related compounds which are stable in the form of aqueous solutions and have good biological activities are obtainable by providing various amino acid residues instead of the following moiety

$$-NH\overset{\overset{OH}{|}}{C}HCO-$$

at the 10- to 12-positions of spergualin.

This invention will be described in more detail below. In the spergualin-related compounds of the general formula (I), $R_1$ represents a hydrogen atom, a hydroxyl group, or an aliphatic acyloxy group with 1 to 10 carbon atoms, such as a formyloxy, acetoxy, propionyloxy, butanoyloxy, pentanoyloxy, hexanoyloxy, heptanoyloxy, octanoyloxy, nonanoyloxy, decanoyloxy group, preferably a lower alkanoyloxy group with 1 to 4 carbon atoms. $R_2$ represents a residue of an α- or ω-amino acid as they are mentioned below,

remaining after removing one of the hydrogen atoms from the α- or ω-amino group or the hydroxyl group from the α-carboxyl group.

Such a residue of the amino acid is hereinafter referred to as the amino acid residue. Said amino acid residue, if it has an optically active carbon atom, may be any of the L-, D- and DL-types. $R_2$ represents groups which have the following formula IV

$$-NH\overset{X}{\overset{|}{C}}H-(CH_2)_n-CO-$$

wherein X represents a hydrogen atom or a straight-chain or branched alkyl group with 1 to 6 carbon atoms, said alkyl group optionally having as a substituent a hydroxyl, carboxyl, lower alkoxycarbonyl, guanidino, phenyl or imidazole group; and n denotes an integer of 0 to 2, and groups which are expressed by the following formula

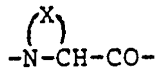

(V)

wherein X represents an alkylene group with up to three carbon atoms. Examples of amino acids corresponding to these groups are α-amino acids such as glycine, alanine, α-aminobutyric acid, proline, valine, norvaline, isoleucine, alloisoleucine, leucine, norleucine, serine, homoserine, threonine, allothreonine, aspartic acid, glutamic acid, arginine, phenylalanine, and histidine, and amino acids such as β-alanine and γ-aminobutyric acid.

In the compounds of the general formula (I), m denotes an integer of 4 to 6. If a substituent is present at the 15-position of the compounds, its configuration may be any of the S-, R- and RS-types.

These compounds of the general formula (I) in which m denotes 4 or 6 and the amino acid residue is a residue of glycine, serine, β-alanine, γ-aminobutanoyl, arginine or phenylalanine, are preferred because of their excellent biological activities. Typical examples of the spergualin-related compounds of the general formula (I) are listed below.

10-[N-(7-guanidinoheptanoyl)glycyl]-1,5,10-triazadecane
10-[N-(7-guanidino-3-hydroxyheptanoyl)glycyl]-1,5,10-triazadecane
10-[N-(7-guanidino-3-acetoxyheptanoyl)glycyl]-1,5,10-triazadecane
10-[N-(7-guanidino-3-propionyloxyheptanoyl)glycyl]-1,5,10-triazadecane
10-[N-(7-guanidino-3-butanoyloxyheptanoyl)glycyl]-1,5,10-triazadecane
10-[N-(9-guanidinononanoyl)glycyl]-1,5,10-triazadecane
10-[N-(9-guanidino-3-hydroxynonanoyl)glycyl]-1,5,10-triazadecane
10-[N-(7-guanidinoheptanoyl)-L-, D- and DL-seryl]-1,5,10-triazadecane
10-[N-(7-guanidino-3-hydroxyheptanoyl)-L-, D- and DL-seryl]-1,5,10-triazadecane
10-[N-(7-guanidino-3-acetoxyheptanoyl)-L-, D- and DL-seryl]-1,5,10-triazadecane
10-[N-(7-guanidino-3-propionyloxyheptanoyl)-L-, D- and DL-seryl]-1,5,10-triazadecane
10-[N-(7-guanidino-3-butanoyloxyheptanoyl)-L-, D- and DL-seryl]-1,5,10-triazadecane
10-[N-(9-guanidinononanoyl)-L-, D- and DL-seryl]-1,5,10-triazadecane
10-[N-(9-guanidino-3-hydroxynonanoyl)-L-, D- and DL-seryl]-1,5,10-triazadecane
10-[N-(7-guanidinoheptanoyl)-L-, D- and DL-alanyl]-1,5,10-triazadecane
10-[N-(7-guanidino-3-hydroxyheptanoyl)-L-, D- and DL-alanyl]-1,5,10-triazadecane
10-[N-(7-guanidino-3-acetoxyheptanoyl)-L-, D- and DL-alanyl]-1,5,10-triazadecane
10-[N-(7-guanidino-3-propionyloxyheptanoyl)-L-, D- and DL-alanyl]-1,5,10-triazadecane
10-[N-(7-guanidino-3-butanoyloxyheptanoyl)-L-, D- and DL-alanyl]-1,5,10-triazadecane
10-[N-(9-guanidinononanoyl)-L-, D- and DL-alanyl]-1,5,10-triazadecane
10-[N-(9-guanidino-3-hydroxynonanoyl)-L-, D- and DL-alanyl]-1,5,10-triazadecane
10-[N-(7-guanidinoheptanoyl)-β-alanyl]-1,5,10-triazadecane
10-[N-(7-guanidino-3-hydroxyheptanoyl)-β-alanyl]-1,5,10-triazadecane
10-[N-(7-guanidino-3-acetoxyheptanoyl)-β-alanyl]-1,5,10-triazadecane
10-[N-(7-guanidino-3-propionyloxyheptanoyl)-β-alanyl]-1,5,10-triazadecane
10-[N-(7-guanidino-3-butanoyloxyheptanoyl)-β-alanyl]-1,5,10-triazadecane
10-[N-(9-guanidinononanoyl)-β-alanyl]-1,5,10-triazadecane
10-[N-(9-guanidino-3-hydroxynonanoyl)-β-alanyl]-1,5,10-triazadecane
10-[N-(7-guanidinoheptanoyl)-γ-aminobutanoyl]-1,5,10-triazadecane
10-[N-(7-guanidino-3-hydroxyheptanoyl)-γ-aminobutanoyl]-1,5,10-triazadecane
10-[N-(7-guanidino-3-acetoxyheptanoyl)-γ-aminobutanoyl]-1,5,10-triazadecane
10-[N-(7-guanidino-3-propionyloxyheptanoyl)-γ-aminobutanoyl]-1,5,10-triazadecane
10-[N-(7-guanidino-3-butanoyloxyheptanoyl)-γ-aminobutanoyl]-1,5,10-triazadecane
10-[N-(9-guanidinononanoyl)-γ-aminobutanoyl]-1,5,10-triazadecane
10-[N-(9-guanidino-3-hydroxynonanoyl)-γ-aminobutanoyl]-1,5,10-triazadecane
10-[N-(7-guanidinoheptanoyl)-L-, D- and DL-α-aminobutanoyl]-1,5,10-triazadecane
10-[N-(7-guanidino-3-hydroxyheptanoyl)-L-, D- and DL-α-aminobutanoyl]-1,5,10-triazadecane

10-[N-(7-guanidino-3-acetoxyheptanoyl)-L-, D- and DL-α-aminobutanoyl]-1,5,10-triazadecane
10-[N-(9-guanidinononanoyl)-L-, D- and DL-α-aminobutanoyl]-1,5,10-triazadecane
10-[N-(7-guanidinoheptanoyl)-L-, D- and DL-propyl]-1,5,10-triazadecane
10-[N-(7-guanidino-3-hydroxyheptanoyl)-L-, D- and DL-propyl]-1,5,10-triazadecane
10-[N-(7-guanidino-3-acetoxyheptanoyl)-L-, D- and DL-propyl]-1,5,10-triazadecane
10-[N-(9-guanidinononanoyl)-L-, D- and DL-propyl]-1,5,10-triazadecane
10-[N-(7-guanidinoheptanoyl)-L-, D- and DL-valyl]-1,5,10-triazadecane
10-[N-(7-guanidino-3-hydroxyheptanoyl)-L-, D- and DL-valyl]-1,5,10-triazadecane
10-[N-(7-guanidino-3-acetoxyheptanoyl)-L-, D- and DL-valyl]-1,5,10-triazadecane
10-[N-(9-guanidinononanoyl)-L-, D- and DL-valyl]-1,5,10-triazadecane
10-[N-(7-guanidinoheptanoyl)-L-, D- and DL-isoleucyl]-1,5,10-triazadecane
10-[N-(7-guanidino-3-hydroxyheptanoyl)-L-, D- and DL-isoleucyl]-1,5,10-triazadecane
10-[N-(7-guanidino-3-acetoxyheptanoyl)-L-, D- and DL-isoleucyl]-1,5,10-triazadecane
10-[N-(9-guanidinononanoyl)-L-, D- and DL-isoleucyl]-1,5,10-triazadecane
10-[N-(7-guanidinoheptanoyl)-L-, D- and DL-leucyl]-1,5,10-triazadecane
10-[N-(7-guanidino-3-hydroxyheptanoyl)-L-, D- and DL-leucyl]-1,5,10-triazadecane
10-[N-(7-guanidino-3-acetoxyheptanoly)-L-, D- and DL-leucyl]-1,5,10-triazadecane
10-[N-(9-guanidinononanlyl)-L-, D- and DL-leucyl]-1,5,10-triazadecane
10-[N-(7-guanidinoheptanoyl)-L-, D- and DL-homoseryl]-1,5,10-triazadecane
10-[N-(7-guanidino-3-hydroxyheptanoyl)-L-, D- and DL-homoseryl]-1,5,10-triazadecane
10-[N-(7-guanidino-3-acetoxyheptanoyl)-L-, D- and DL-homoseryl]-1,5,10-triazadecane
10-[N-(9-guanidinononanoyl)-L-, D- and DL-homoseryl]-1,5,10-triazadecane
10-[N-(7-guanidinoheptanoyl)-L-, D- and DL-threonyl]-1,5,10-triazadecane
10-[N-(7-guanidino-3-hydroxyheptanoyl)-L-, D- and DL-threonyl]-1,5,10-triazadecane
10-[N-(7-guanidino-3-acetoxyheptanoyl)-L-, D- and DL-threonyl]-1,5,10-triazadecane
10-[N-(9-guanidinononanoyl)-L-, D- and DL-threonyl]-1,5,10-triazadecane
10-[N-(7-guanidinoheptanoyl)-L-, D- and DL-aspartyl]-1,5,10-triazadecane
10-[N-(7-guanidino-3-hydroxyheptanoyl)-L-, D- and DL-aspartyl]-1,5,10-triazadecane
10-[N-(7-guanidino-3-acetoxyheptanoyl)-L-, D- and DL-aspartyl]-1,5,10-triazadecane
10-[N-(9-guanidinononanoyl)-L-, D- and DL-aspartyl]-1,5,10-triazadecane
10-[N-(7-guanidinoheptanoyl)-L-, D- and DL-glutamyl]-1,5,10-triazadecane
10-[N-(7-guanidino-3-hydroxyheptanoyl)-L-, D- and DL-glutamyl]-1,5,10-triazadecane
10-[N-(7-guanidino-3-acetoxyheptanoyl)-L-, D- and DL-glutamyl]-1,5,10-triazadecane
10-[N-(9-guanidinononanoyl)-L-, D- and DL-glutamyl]-1,5,10-triazadecane
10-[$N^α$-(7-guanidinoheptanoyl)-L-, D- and DL-arginyl]-1,5,10-triazadecane
10-[$N^α$-(7-guanidino-3-hydroxyheptanoyl)-L-, D- and DL-arginyl]-1,5,10-triazadecane
10-[$N^α$-(7-guanidino-3-acetoxyheptanoyl)-L-, D- and DL-arginyl]-1,5,10-triazadecane
10-[$N^α$-(9-guanidinononanoyl)-L-, D- and DL-arginyl]-1,5,10-triazadecane
10-[N-(7-guanidinoheptanoyl)-L-, D- and DL-phenylalanyl]-1,5,10-triazadecane
10-[N-(7-guanidino-3-hydroxyheptanoyl)-L-, D- and DL-phenylalanyl]-1,5,10-triazadecane
10-[N-(7-guanidino-3-acetoxyheptanoyl)-L-, D- and DL-phenylalanyl]-1,5,10-triazadecane
10-[N-(9-guanidinononanoyl)-L-, D- and DL-phenylalanyl]-1,5,10-triazadecane
10-[$N^α$-(7-guanidinoheptanoyl)-L-, D- and DL-histidyl]-1,5,10-triazadecane
10-[$N^α$-(7-guanidino-3-hydroxyheptanoyl)-L-, D- and DL-histidyl]-1,5,10-triazadecane
10-[$N^α$-(7-guanidino-3-acetoxyheptanoyl)-L-, D- and DL-histidyl]-1,5,10-triazadecane
10-[$N^α$-(9-guanidinononanoyl)-L-, D- and DL-histidyl]-1,5,10-triazadecane

The spergualin-related compounds of the general formula (I) form salts with acids. The acids for forming their salts may be anyl inorganic or organic acids which are non-toxic. The inorganic acids are not restricted in their types, but their preferred examples are hydrochloric acid, sulfuric acid, nitric acid and phosphoric acid. The types of the organic acids are also unrestricted, but preferably, they include, for example, acetic acid, propionic acid, succinic acid, fumaric acid, maleic acid, malic acid, tartaric acid, glutaric acid, citric acid, benenesulfonic acid, toluenesulfonic acid, methanesulfonic acid, ethanesulfonic acid, propanesulfonic acid, aspartic acid and glutamic acid.

The spergualin-related compounds of the general formula (I) in accordance with the present invention are prepared by removing protective groups from the protected spergualin-related compounds of the general formula (II) by means of known methods, such as reduction, hydrolysis and acid decomposition, Table 1 shows typical examples of the protective groups in the protected spergualin-related compounds, and examples of methods for removing these protective groups. In Table 1, the disclosures concerned with the protective group for each of carboxyl, hydroxyl, imidazole and guanidino groups are related to $R'_2$ which has any of these groups as a protected substituent, and to methods of removing the protective group from the protected substituent.

In Table 1, the symbol + shows the removability of the protective group by the indicated method. The symbol − shows that the protective group cannot be removed by the indicated method. The symbol ± shows partial removal or decomposition, indicating that the method at issue is not very suitable for the protective group.

The protective groups that can be used in this invention are not limited to those described in Table 1. For instance, there can be used all of those disclosed in Shiro Akabori, Takeo Kaneko and Kozo Narita: Tanpakushitsu Kagaku (Protein Chemistry) 1; Amino Acids. Peptides (Kyoritsu Shuppan, 1969); Nobuo Izumiya: Peptide Gosei (Peptide Synthesis) (Maruzen, 1975); E. Schröder and K. Lübke: The Peptides Academic Press (New York, 1965); E. Wünsch Methoden der Organischen Chemie (Houben. Weyl), Synthese von Peptiden. Georg Thieme Verlag Stuttgart (1974); M. Bodomszky and M. A. Ondetti: Peptide Synthesis. Interscience Publishers (New York, 1976).

From the reaction mixture deprived of the protective groups is then isolated the spergualin-related compound of the general formula (I). If the protective groups have been removed by catalytic reduction with palladium black, for example, the isolation is carried out by removing the catalyst by filtration, concentrating the filtrate under reduced pressure, and purifying the residue by a known purification method using CM-Sephadex® ($Na^+$) and Sephadex® LH—20 (T. Takeuchi et al., J. Antibiotics 34, 1619 (1981)). If the protective groups have been removed by using trifluoroacetic acid, on the other hand, the desired compound can be isolated by concentrating the reaction mixture under reduced pressure, and purifying the residue by the same known purification method.

The above-mentioned purification method enables the spergualin-related compound of the general formula (I) to be obained as a hydrochloride. The resulting hydrochloride can be converted into other salts in the following way: The hydrochloride is dissolved in water, and the aqueous solution is passed through a strongly basic ion exchange resin. Fractions containing the desired product to be referred (to as active fractions) are collected, and an acid to form the desired salt or an aqueous solution of the acid or a solution of the acid in a hydrophilic organic solvent such as methanol, ethanol, acetone, tetrahydrofuran or dioxane, is added to the collected fractions for neutralization. The neutralized liquid is dried under reduced pressure, or if it contains the organic solvent, the organic solvent is removed by distillation under reduced pressure, followed by freeze-drying the residue.

Alternatively, an aqueous solution of silver hydroxide or silver oxide is added to the hydrochloride of the compound of the general formula (I) to neutralize the hydrochloric acid. The insoluble silver chloride is removed by filtration, followed by adding a desired acid to the filtrate to form a salt, which is then freeze-dried.

The product obtained in the above-mentioned manner may include a hydrate depending on the conditions for treatment.

The protected spergualin-related compounds of the general formula (II), the starting materials of the present invention, are novel compounds and can be synthesized in the following way.

1,5,10-Diprotected-1,5,10-triazadecanes of the general formula (VI)

$$H_2N(CH_2)_4\overset{\overset{R_3}{|}}{N}(CH_2)_3NH-R_4 \qquad (VI)$$

wherein $R_3$ and $R_4$ are as defined earlier, are condensed with N-protected — or -amino acids of the formula $R_5OH$ wherein $R_5$ represents a residue of an α- or ω-amino acid corresponding to those defined above under $R_2$ remaining after removing the hydroxyl group from the α-carboxyl group, the amino group of the α- or ω-amino acid being protected with a protective group different from the one represented by $R_3$ or $R_4$, to form 10-(N-protected aminoacyl)-1,5-diprotected-1,5,10-triazadecanes of the general formula (VII)

$$R_5-NH(CH_2)_4\overset{\overset{R_3}{|}}{N}(CH_2)_3-NH-R_4 \qquad (VII)$$

wherein $R_3$, $R_4$ and $R_5$ are as defined earlier.

The protective group for the amino group in the amino acid residue $R_5$ of the resulting compounds is removed by customary methods to obtain 10-aminoacyl-1,5-diprotected-1,5,10-triazadecanes of the general formula (VIII)

$$H-R_2'-NH(CH_2)_4\overset{\overset{R_3}{|}}{N}(CH2)_3NH-R_4 \qquad (VIII)$$

wherein $R_2'$, $R_3$ and $R_4$ are as defined earlier.

Then, the resulting compounds are condensed with -guanidino fatty acids of the general formula (IX)

$$H_2N\underset{\underset{NH}{\|}}{C}NH(CH_2)_m\underset{\underset{R_1}{|}}{C}HCH_2COOH \qquad (IX)$$

wherein $R_1$ and m are as defined earlier, to obtain the protected spergualin-related compounds of the general formula (II).

The condensation between the compounds of the general formula (IX) can be performed by customary methods for use in the formation of peptide bonds. Examples of these methods are the carbodiimide method using dicyclohexylcarbodiimide, or 1-ethyl-8-(3-dimethylaminopropyl)-carbodiimide; the azide method involving hydrazides; the mixed acid anhydride method using ethyl chlorocarbonate or isobutyl chlorocarbonate; the active ester method involving cyanomethyl ester, vinyl ester, substituted and unsubstituted phenyl ester, thiophenyl ester, or hydroxysuccinimide ester; the O-acylhydroxylamine derivative method using acetoxime or cyclohexanone oxime; and the N-acyl compound method using carbodiimidazole or the like. Solvents for use in the condensation may be those to be used in forming peptide bonds. Examples of the solvents are ethers such as diethyl ether, tetrahydrofuran, and dioxane; esters such as ethyl acetate; ketones such as acetone and methyl ethyl ketone; halogenated hydrocarbons such as methylene chloride and chloroform; amides such as dimethylformamide and dimethylacetamide; and nitriles such as acetonitrile.

The 1,5-diprotected-1,5,10-triazadecanes of the general formula (III), the raw materials in the above reactions, can be synthesized in the following manner:

Monoamino protected derivatives of 1,4-butanediamine of the following formula (X)

$$R_6HN(CH_2)_4NH_2 \qquad (X)$$

wherein $R_6$ represents an amino-protecting group different from that represented by $R_4$, are condensed by custumary methods with amino-protected derivatives of 3-halopropanamines of the general formula (XI)

$$X(CH_2)_3NHR_4 \qquad (XI)$$

wherein $R_4$ represents an amino-protecting group identical to that represented by the $R_4$ previously defined, and X represents a halogen atom, to form compounds of the formula (XII)

$$R_6HN(CH_2)_4NH(CH_2)_3NHR_4 \qquad (XII)$$

wherein $R_4$ and $R_6$ are different and each represent an amino-protecting group.

The remaining imino group is protected with an amino-protecting group which is represented by $R_3$, which can be eliminated by the same method as for $R_4$, and which is different from the amino-protecting group represented by $R_6$. Then, the amino-protecting group expressed by $R_6$ is selectively eliminated to obtain the 1,5-diprotected-1,5,10-triazadecanes of the general formula (VI).

The 1,5-diprotected-1,5,10-triazadecanes of the general formula (VI) in which the protective groups $R_3$ and $R_4$ are identical to each other can be synthesized in the following manner:

1-(4-aminobutyl)-hexahydropyrimidine of the formula (XIII)

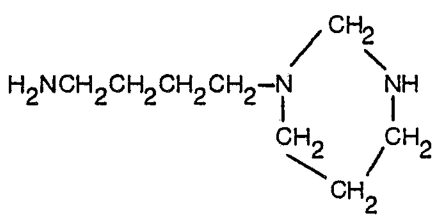

(XIII)

is subjected to the action of N-ethoxycarbonylphthalimide to form 1-(4-phthaliminobutyl)-hexahydropyrimidine of the formula (XIV)

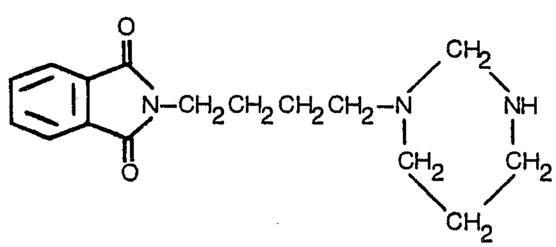

(XIV)

The resulting compound is hydrolyzed under acidic conditions to form 10-phthalyl-1,5,10-triazadecane of the formula (XV)

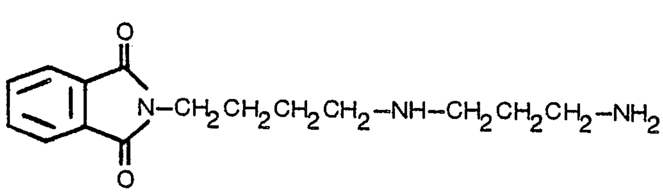

(XV)

The compound thus formed is subjected to the action of an agent which protects an amino group by changing it into an R-containing form, whereby 10-phthalyl-1,5,10-diprotected-1,5,10-triazadecanes of the formula (XVI)

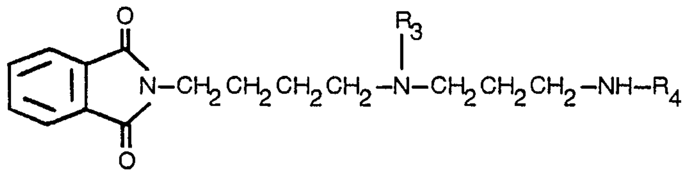

(XVI)

wherein $R_3$ and $R_4$ are identical and each represent a protective group, are formed. The phthalyl group is removed from the resulting compounds by customary methods to obtain 1,5-diprotected-1,5,10-triazadecanes of the general formula (VI) in which $R_3$ and $R_4$ are identical.

The amino-protecting groups may be those which have been hitherto used widely in peptide synthesis. The amino-protecting groups used as $R_6$, however, are those which should be removed selectively, unlike the amino-protecting groups $R_3$ and $R_4$ which are to remain intact. The ω-guanidino fatty acids of the general formula (IX) can be synthesized, for instance, in the following way:

These compounds in which $R_1$ is a hydroxyl group and m is 4 are known compounds as described in The Journal of Antibiotics, Vol. 36, Page 1623 (1981), and can be obtained by the hydroxlysis of spergualin. The compound in with $R_1$ is an acyloxy group and m is 4 can be obtained by acylating the same compounds, having a hydroxyl group as $R_1$, by common methods. The compounds in which $R_1$ represents a hydrogen atom and m denotes 4 to 6 are disclosed in British Patent 1153. 424 and can be obtained by converting the amino group of the corresponding ω-amino fatty acids into a guanidino group by ordinary methods.

Table 1 Protective groups for functional groups of amino acids and methods of removing them

| Abbreviation | $H_2$/pd | Na/$NH_3$ | HBr/AcOH | HCL | AcOH | $CF_3COOH$ | $NH_2NH_2$ | NaOH | $NH_4OH$ | HF |
|---|---|---|---|---|---|---|---|---|---|---|
| Removing agent | Hydrogen in the presence of palladium | Metalic sodium | Hydrogen bromide | Hydrogen chloride | Acetic acid | Trifluoroacetic acid | Hydrazine | Sodium hydroxide | Ammonia | Liquid hydrogen fluoride |
| Solvent | Lower alcohol dimethylformamide Acetic Acid | Liquid ammonia | Acetic acid | Ethyl acetate lower alcohol acetic acid | Acetic acid | Trifluoroacetic acid | Lower alcohol | Water lower alcohol | Water lower alcohol | Liquid hydrogen fluoride |
| Temperature | room temp. to 50°C | -30°C or lower | 0 to 50°C | 0°C to room temp. | 0°C to b.p. | 0°C to room temp. | room temp. to b.p. | 0 to 50°C | 0 to 50°C | 20°C or lower |
| Pressure | atmospheric pressure to 100 $kg/cm^2$ | atmospheric pressure | atmospheric pressure | atmospheric pressure | atmospheric press. | atmospheric pressure | atmospheric pressure | atmospheric press. | atmospheric pressure | atmospheric pressure |
| $C_6H_5CH_2OCO-$ | + | + | + | ± | − | ± | − | − | − | + |
| $(CH_3)_3COCO-$ | − | − | + | + | + | + | − | − | − | + |
| $(C_6H_5)$ C | + | + | + | + | + | + | − | − | − | |
| 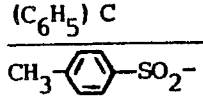 | − | + | − | − | − | − | − | − | − | − |
| HCO- | − | − | − | + | − | − | − | − | − | − |

Table 1 (Cont.)

| Abbreviation (Amino Protecting Group) | $H_2$/pd | Na/$NH_3$ | HBr/AcOH | HCl | AcOH | $CF_3COOH$ | $NH_2NH_2$ | NaOH | $NH_4OH$ | HF |
|---|---|---|---|---|---|---|---|---|---|---|
| 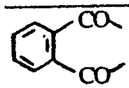 | − | ± | − | − | − | − | + | ± | ± | − |
| $CF_3CO-$ | − | + | − | + | − | − | + | + | + | − |
| 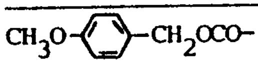 | + | + | + | + | − | + | − | − | − | |
| 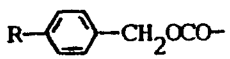 | + | + | + | + | − | + | − | − | − | |
| $C_6H_5CH_2-$ | + | + | − | − | − | − | − | − | − | |
| 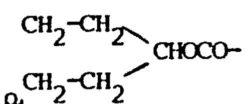 | − | | + | − | | − | − | | | + |
| $(C_6H_5)_2CHOCO-$ | + | + | + | + | | + | | − | | |
| 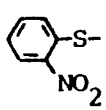 | | | + | + | | + | | | | |
| $(C_6H_5)_3C-S-$ | | | | + | | | | | | |
| $(CH_3)_2CHOCO-$ | − | − | ± | − | | − | | | | + |

a) Cl, Br, $NO_2$, 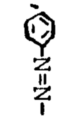 , $CH_3$- 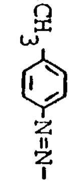

| | Abbreviation | $H_2/pd$ | $Na/NH_3$ | HBr/AcOH | HCl | AcOH | $CF_3COOH$ | $NH_2NH_2$ | NaOH | $NH_4OH$ | HF |
|---|---|---|---|---|---|---|---|---|---|---|---|
| Removal method | Removing agent | Hydrogen /presence of palladium | Metalic sodium | Hydrogen bromide | Hydrogen chloride | Acetic acid | Trifluoroacetic acid<br>-- | Hydrazine | Sodium hydroxide | Ammonia | Liquid hydrogen fluoride |
| | Solvent | Lower alcohol dimethylformamide | Liquid ammonia | Acetic acid | Ethyl acetate Lower alcohol Acetic acid | Acetic acid | Trifluoroacetic acid | Lower alcohol | Water Lower alcohol | Water Lower alcohol | Liquid hydrogen fluoride |
| | Temperature | room temp. to 50°C | -30°C or lower | 0 to 50°C | 0°C to room temp. | 0°C to b.p. | 0°C to room temp. | room temp. to b.p. | 0 to 50°C | 0 to 50°C | 20°C or lower |
| | Pressure | atmospheric press. to $100kg/cm^2$ | atmospheric press. | atmospheric press. | atmospheric press. | atmospheric press. | atmospheric press. | atmospheric press. | atmospheric press. | atmospheric press. | atmospheric pressure |
| Carboxyl-protect. group | Lower alkyl | - | + | - | + | - | - | - | + | + | - |
| | $(CH_3)_3C-$ | - | + | + | + | + | + | - | + | - | + |
| | $C_6H_5CH_2-$ | + | + | + | + | - | + | - | + | | + |
| | $CH_3O-$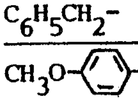$-CH_2-$ | + | | + | + | | + | | + | | |
| | $NO_2-$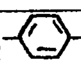$-CH_2-$ | + | + | - | - | - | - | | + | | $\pm$ |

(Cont.)

| | Abbreviation | $H_2pd$ | $Na/NH_3$ | HBr/AcOH | HCl | AcOH | $CF_3COOH$ | $NH_2NH_2$ | NaOH | $NH_4OH$ | HF |
|---|---|---|---|---|---|---|---|---|---|---|---|
| carboxyl-protect. groups | $(C_6H_5)_2CH-$ | + | | + | + | | + | | + | | |
| | $(CH_3)_2CH-$ | − | − | − | − | | − | | + | | + |
| | 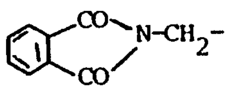 | | | + | | | | + | + | | |
| | $CH_3CO-$ | − | | − | − | | − | + | + | + | − |
| | $C_6H_5CO-$ (phenolic) | − | | | | | | | + | | |
| | $(CH_3)_3C-$ | − | − | + | + | | + | − | + | | + |
| | $C_6H_5CH_2-$ | + | + | + | − | − | − | − | − | − | + |
| hydroxyl-protecting groups | 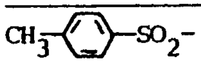 (phenolic) | − | + | − | − | − | − | | − | | |
| | 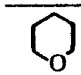 | − | | | + | + | | | | | |
| | $C_6H_5CH_2OCO-$ (phenolic) | + | + | + | | | + | | | | |
| | $(C_6H_5)_3C-$ | + | | + | + | | + | | | | |

| | Abbreviation | $H_2$/pd | Na/$NH_3$ | HBr/AcOH | HCl | AcOH | $CF_3COOH$ | $NH_2NH_2$ | NaOH | $NH_4OH$ | HF |
|---|---|---|---|---|---|---|---|---|---|---|---|
| | Removing agent | Hydrogen in the presence of palladium | Metallic sodium | Hydrogen bromide | Hydrogen chloride | Acetic acid | Trifluoro-acetic acid | Hydrazine | Sodium hydro-xide | Ammonia | Liquid hydrogen fluoride |
| | Solvent | Lower alcohol dimethyl-formamide Acetic acid | Liquid ammonia | Acetic acid | Ethyl acetate Lower alcohol acetic acid | Acetic acid | Trifluoro-acetic acid | Lower alcohol | Water Lower alcohol | Water Lower alcohol | Liquid hydrogen fluoride |
| Removal method | Temperature | room temp. to 50°C | -30°C or lower | 0 to 50°C | 0°C to room temp. | 0°C to b.p. | 0°C to room temp. | room temp. to b.p. | 0 to 50°C | 0 to 50°C | 20°C or lower |
| | Pressure | atmos-pheric pressure to 100 $kg/cm^2$ | atmos-pheric press. | atmos.-pheric press. | atmos-pheric press. | atmos.-pheric press. | atmos.-pheric press. | atmos - pheric press. | atmos.-pheric press. | atmos.-pheric press. | atmos.-pheric pressure |
| Nitrogen prot. group | $C_6H_5CH_2OCO-$ | + | + | + | − | | + | | + | | + |
| | $C_6H_5CH_2-$ | ± | + | − | − | − | − | − | − | | |
| | $(C_6H_5)_3C-$ | | + | + | + | | + | | − | | |
| | $NO_2-$ | + | ± | − | − | − | − | − | − | | + |
| Guanidino prot. group | 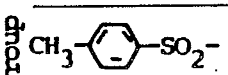 | − | + | − | − | − | − | − | − | | |
| | 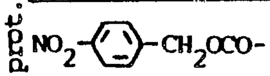 | + | + | + | − | | + | | + | | + |
| | $(CH_3)_3COCO-$ | − | − | + | + | | + | | − | | + |

The protected spergualin compounds of the general formula II can also be synthesized by condensing acid salts of ω-guanidinoacylamino acids of the general formula (XVII)

$$H_2N\underset{\underset{NH}{\|}}{C}NH(CH_2)_m\underset{\underset{R_1}{|}}{C}HCH_2CO-R'_2-OH \qquad (XVII)$$

wherein $R_1$, $R'_2$ and m are as defined previously, with protected 1,5,10-triazadecanes of the general formula (VI)

$$H_2N(CH_2)_4\overset{\overset{R_3}{|}}{N}(CH_2)_3NH-R_4 \qquad (VI)$$

The ω-guanidinoacylamino acids of the general formula (XVII) are novel compounds, and can be obtained by condensing acid salts of the ω-guanidino fatty acids of the general formula (IX) with α- or ω-amino acids of the general formula (XVIII)

$$H—R'_2—O—R_7 \qquad (XVIII)$$

wherein $R'_2$ is as defined above, and $R_7$ represents a protective group for the α-carboxyl group of the α- or ω-amino acid, or a hydrogen atom, to form acid salts of ω-guanidinoacylamino acids of the general formula (XIX)

$$H_2N\underset{\underset{NH}{\|}}{C}NH(CH_2)_m\underset{\underset{R_1}{|}}{C}HCH_2CO-R'_2-O-R_7 \qquad (XIX)$$

wherein m, $R_1$, $R'_2$ and $R_7$ are as defined above, and if $R_7$ is the protective group, further removing it by customary methods. The condensation reaction between the ω-guanidinoacylamino acids of the general formula (XVII) and the protected 1,5,10-triazadecanes of the general formula (VI) can be carried out in the same way as for the condensation between the compounds of the general formula (VIII) and the compounds of the general formula (IX).

Typical examples of the protected spergualin-related compounds of the general formula (V) are listed below:

10-[N-(7-guanidinoheptanoyl)glycyl]-1,5-dibenzyloxycarbonyl-1,5,10-triazadecane
10-[N-(7-guanidinoheptanoyl)glycyl]-1,5-tert-butoxycarbonyl-1,5,10-triazadecane
10-[N-(7-guanidino-3-hydroxyheptanoyl)glycyl]-1,5-dibenzyloxycarbonyl-1,5,10-triazadecane
10-[N-(7-guanidino-3-hydroxyheptanoyl)glycyl]-1,5-di-tert-butoxycarbonyl-1,5,10-triazadecane
10-[N-(7-guanidino-3-acetoxyheptanoyl)glycyl]-1,5-dibenzyloxycarbonyl-1,5,10-triazadecane
10-[N-(7-guanidino-3-acetoxyheptanoyl)glycyl]-1,5-di-tert-butoxycarbonyl-1,5,10-triazadecane
10-[N-(7-guanidino-3-propionyloxyheptanoyl)glycyl]-1,5-dibenzyloxycarbonyl-1,5,10-triazadecane
10-[N-(7-guanidino-3-propionyloxyheptanoyl)glycyl]-1,5-di-tert-butoxycarbonyl-1,5,10-triazadecane
10-[N-(7-guanidino-3-butanoyloxyheptanoyl)glycyl]-1,5-dibenzyloxycarbonyl-1,5,10-triazadecane
10-[N-(7-guanidino-3-butanoyloxyheptanoyl)glycyl]-1,5-di-tert-butoxycarbonyl-1,5,10-triazadecane
10-[N-(9-guanidinononanoyl)glycyl]-1,5-dibenzyloxycarbonyl-1,5,10-triazadecane
10-[N-(9-guanidinononanoyl)glycyl]-1,5-di-tert-butoxycarbonyl-1,5,10-triazadecane
10-[N-(9-guanidino-3-hydroxynonanoyl)glycyl]-1,5-dibenzyloxycarbonyl-1,5,10-triazadecane
10-[N-(9-guanidino-3-hydroxynonanoyl)glycyl]-1,5-di-tert-butoxycarbonyl-1,5,10-triazadecane
10-[N-(7-guanidinoheptanoyl)-L-, D- and DL-seryl]-1,5-dibenzyloxycarbonyl-1,5,10-triazadecane
10-[N-(7-guanidinoheptanoyl)-L-, D- and DL-seryl]-1,5-di-tert-butoxycarbonyl-1,5,10-triazadecane
10-[N-(7-guanidinoheptanoyl)-O-benzyl-L-, D- and DL-seryl]-1,5-dibenzyloxycarbonyl-1,5,10-triazadecane
10-[N-(7-guanidinoheptanoyl)-O-tert-butyl-L-, D- and DL-seryl]-1,5-di-tert-butoxycarbonyl-1,5,10-triazadecane
10-[N-(7-guanidino-3-hydroxyheptanoyl)-L-, D- and DL-seryl]-1,5-dibenzyloxycarbonyl-1,5,10-triazadecane
10-[N-(7-guanidino-3-hydroxyheptanoyl)-L-, D- and DL-seryl]1,5-di-tert-butoxycarbonyl-1,5,10-triazadecane
10-[N-(7-guanidino-3-hydroxyheptanoyl)-O-benzyl-L-, D- and DL-seryl]-1,5-dibenzyloxycarbonyl-1,5,10-triazadecane
10-[N-(7-guanidino-3-hydroxyheptanoyl)-O-tert-butyl-L-, D- and DL-seryl]-1,5-di-tert-butoxycarbonyl-1,5,10-triazadecane
10-[N-(7-guanidino-3-acetoxyheptanoyl)-L-, D- and DL-seryl)-1,5-dibenzyloxycarbonyl-1,5,10-triazadecane

10-[N-(7-guanidino-3-acetoxyheptanoyl)-L-, D- and DL-seryl]-1,5-di-tert-butoxycarbonyl-1,5,10-triazadecane

10-[N-(7-guanidino-3-acetoxyheptanoyl)-O-benzyl-L-, D- and DL-seryl]-1,5-dibenzyloxycarbonyl-1,5,10-triazadecane

10-[N-(7-guanidino-3-acetoxyheptanoyl)-O-tert-butyl-L-, D- and DL-seryl]-1,5-di-tert-butoxycarbonyl-1,5,10-triazadecane

10-[N-(7-guanidino-3-propionyloxyheptanoyl)-L-, D- and DL-seryl]-1,5-dibenzyloxycarbonyl-1,5,10-triazadecane

10-[N-(7-guanidino-3-propionyloxyheptanoyl)-L-, D- and DL-seryl]-1,5-di-tert-butoxycarbonyl-1,5,10-triazadecane

10-[N-(7-guanidino-3-propionyloxyheptanoyl)-O-benzyl-L-, D- and DL-seryl]-1,5-dibenzyloxycarbonyl-1,5,10-triazadecane

10-[N-(7-guanidino-3-propionyloxyheptanoyl)glycyl]-O-tert-butyl-L-, D- and DL-seryl]-1,5-di-tert-butoxycarbonyl-1,5,10-triazadecane

10-[N-(7-guanidino-3-butanoyloxyheptanoyl)-L-, D- and DL-seryl]-1,5-dibenzyloxycarbonyl-1,5,10-triazadecane

10-[N-(7-guanidino-3-butanoyloxyheptanoyl)-L-, D- and DL-seryl]-1,5-di-tert-butoxycarbonyl-1,5,10-triazadecane

10-[N-(7-guanidino-3-butanoyloxyheptanoyl)-O-benzyl-L-, D- and DL-seryl]-1,5-dibenzyloxycarbonyl-1,5,10-triazadecane

10-[N-(7-guanidino-3-butanoyloxyheptanoyl)-O-tert-butyl-L-, D- and DL-seryl]-1,5-di-tert-butoxycarbonyl-1,5,10-triazadecane

10-[N-(9-guanidinononanoyl)-L-, D- and DL-seryl]-1,5-dibenzyloxycarbonyl-1,5,10-triazadecane

10-[N-(9-guanidinononanoyl)-L-, D- and DL-seryl]-1,5-di-tert-butoxycarbonyl-1,5,10-triazadecane

10-[N-(9-guanidinononanoyl)-O-benzyl-L-, D- and DL-seryl]-1,5-dibenzyloxycarbonyl-1,5,10-triazadecane

10-[N-(9-guanidinononanoyl)-O-tert-butyl-L-, D- and DL-butyl-seryl]-1,5-di-tert-butoxycarbonyl-1,5,10-triazadecane

10-[N-(9-guanidino-3-hydroxynonanoyl)-L-, D- and DL-seryl]-1,5-dibenzyloxycarbonyl-1,5,10-triazadecane

10-[N-(9-guanidino-3-hyroxynonanoyl)-O-benzyl-L-, D- and DL-seryl]-1,5-dibenzyloxycarbonyl-1,5,10-triazadecane

10-[N-(7-guanidinoheptanoyl)-L-, D- and DL-alanyl]-1,5-dibenzyloxycarbonyl-1,5,10-triazadecane

10-[N-(9-guanidinononanoyl)-L-, D- and DL-alanyl]-1,5-dibenzyloxycarbonyl-1,5,10-triazadecane

10-[N-(7-guanidinoheptanoyl)-β-alanyl]-1,5-dibenzyloxycarbonyl-1,5,10-triazadecane

10-[N-(7-guanidinoheptanoyl)-β-alanyl]-1,5-di-tert-butoxycarbonyl-1,5,10-triazadecane

10-[N-(7-guanidino-3-hydroxyheptanoyl)-β-alanyl]-1,5-dibenzyloxycarbonyl-1,5,10-triazadecane

10-[N-(7-guanidino-3-hydroxyheptanoyl)-β-alanyl]-1,5-di-tert-butoxycarbonyl-1,5,10-triazadecane

10-[N-(7-guanidino-3-acetoxyheptanoyl)-β-alanyl]-1,5-dibenzyloxycarbonyl-1,5,10-triazadecane

10-[N-(7-guanidino-3-acetoxyheptanoyl)-β-alanyl]-1,5-di-tert-butoxycarbonyl-1,5,10-triazadecane

10-[N-(7-guanidino-3-propionyloxyheptanoyl)-β-alanyl]-1,5-dibenzyloxycarbonyl-1,5,10-triazadecane

10-[N-(7-guanidino-3-butanoyloxyheptanoyl)-β-alanyl]-1,5-dibenzyloxycarbonyl-1,5,10-triazadecane

10-[N-(9-guanidinononanoyl)-β-alanyl]-1,5-dibenzyloxycarbonyl-1,5,10-triazadecane

10-[N-(9-guanidinononanoyl)-β-alanyl]-1,5-di-tert-butoxycarbonyl-1,5,10-triazadecane

10-[N-(9-guanidino-3-hydroxynonanoyl)-β-alanyl]-1,5-dibenzyloxycarbonyl-1,5,10-triazadecane

10-[N-(9-guanidino-3-hydroxynonanoyl)-β-alanyl]-1,5-di-tert-butoxycarbonyl-1,5,10-triazadecane

10-[N-(7-guanidinoheptanoyl)-γ-aminobutanoyl]-1,5-dibenzyloxycarbonyl-1,5,10-triazadecane

10-[N-(7-guanidino-3-hydroxyheptanoyl)-γ-aminobutanoyl]-1,5-dibenzyloxycarbonyl-1,5,10-triazadecane

10-[N-(7-guanidinoheptanoyl)-L-, D- and DL-α-aminobutanoyl]-1,5-dibenzyloxycarbonyl-1,5,10-triazadecane

10-[N-(7-guanidinoheptanoyl)-L-, D- and DL-prolyl]-1,5-dibenzyloxycarbonyl-1,5,10-triazadecane

10-[N-(7-guanidinoheptanoyl)-L-, D- and DL-valyl]-1,5-dibenzyloxycarbonyl-1,5,10-triazadecane

10-[N-(7-guanidinoheptanoyl)-L-, D- and DL-isoleucyl]-1,5-dibenzyloxycarbonyl-1,5,10-triazadecane

10-[N-(7-guanidinoheptanoyl)-L-, D- and DL-leucyl]-1,5-dibenzyloxycarbonyl-1,5,10-triazadecane

10-[N-(7-guanidino-3-hydroxyheptanoyl)glycyl)-L-, D- and DL-leucyl]-1,5-dibenzyloxycarbonyl-1,5,10-triazadecane

10-[N-(7-guanidinoheptanoyl)-L-, D- and DL-homoseryl]-1,5-dibenzyloxycarbonyl-1,5,10-triazadecane

10-[N-(7-guanidinoheptanoyl)-L-, D- and DL-homoseryl]-1,5-di-tert-butoxycarbonyl-1,5,10-triazadecane

10-[N-(9-guanidinononanoyl)-L-, D- and DL-homoseryl]-1,5-dibenzyloxycarbonyl-1,5,10-triazadecane

10-[N-(7-guanidinoheptanoyl)-L-, D- and DL-threonyl]-1,5-dibenzyloxycarbonyl-1,5,10-triazadecane

10-[N-(7-guanidino-3-hydroxyheptanoyl)-L-, D- and DL-threonyl]-1,5-dibenzyloxycarbonyl-1,5,10-triazadecane

10-[N-(9-guanidinononanoyl)-L-, D- and DL-threonyl]-1,5-dibenzyloxycarbonyl-1,5,10-triazadecane

10-[N-(7-guanidinoheptanoyl)-β-benzyl-L-, D- and DL-aspartyl]-1,5-dibenzyloxycarbonyl-1,5,10-triazadecane

10-[N-(7-guanidino-3-hydroxyheptanoyl)-L-, D- and DL-aspartyl]-1,5-dibenzyloxycarbonyl-1,5,10-triazadecane

10-[N-(9-guanidinononanoyl)-L-, D- and DL-aspartyl]-1,5-dibenzyloxycarbonyl-1,5,10-triazadecane

10-[N-(7-guanidinoheptanoyl)-L-, D- and DL-glutamyl]-1,5-dibenzyloxycarbonyl-1,5,10-triazadecane

10-[N-(7-guanidino-3-hydroxyheptanoyl)-L-, D- and DL-glutamyl]-1,5-dibenzyloxycarbonyl-1,5,10-triazadecane

10-[N-(9-guanidinononanoyl)-L-, D- and DL-glutamyl]-1,5-dibenzyloxycarbonyl-1,5,10-triazadecane

10-[$N^α$-(7-guanidinoheptanoyl)-L-, D- and DL-arginyl]-1,5-dibenzyloxycarbonyl-1,5,10-triazadecane

10-[$N^α$-(7-guanidinoheptanoyl)-L-, D- and DL-arginyl]-1,5-di-tert-butoxycarbonyl-1,5,10-triazadecane

10-[$N^α$-(7-guanidinoheptanoyl)-$N^G$-nitro-L-, D- and DL-arginyl]-1,5-dibenzyloxycarbonyl-1,5,10-triazadecane

10-[$N^α$-(7-guanidino-3-hydroxyheptanoyl)-L-, D- and DL-arginyl]-1,5-dibenzyloxycarbonyl-1,5,10-triazadecane

10-[N-(7-guanidinoheptanoyl)-L-, D- and DL-phenylalanyl]-1,5-dibenzyloxycarbonyl-1,5,10-triazadecane

10-[N-(7-guanidinoheptanoyl)-L-, D- and DL-phenylalanyl]-1,5-di-tert-butoxycarbonyl-1,5,10-triazadecane

10-[N-(7-guanidino-3-hydroxyheptanoyl)-L-, D- and DL-phenylalanyl]-1,5-dibenzyloxycarbonyl-1,5,10-triazadecane

10-[N-(7-guanidino-3-acetoxyheptanoyl)-L-, D- and DL-phenylalanyl]-1,5-dibenzyloxycarbonyl-1,5,10-triazadecane

10-[N-(9-guanidinononanoyl)-L-, D- and DL-phenylalanyl]-1,5-dibenzyloxycarbonyl-1,5,10-triazadecane

10-[$N^α$-(7-guanidinoheptanoyl-$N^{im}$-benzyloxycarbonyl-L-, D- and DL-histidyl]-1,5-dibenzyloxycarbonyl-1,5,10-triazadecane

Experiments were conducted on the compounds of this invention to investigate their stability in aqueous solutions, their inhibitory action on the growth of Bacillus subtilis, their in vitro inhibitory action on the proliferation of mouse leukemia L1210 cells, and their in vivo action on the prolongation of life and their toxicity in mice receiving the same cells by transplantation.

Table 2 shows the compounds of this invention that were used in these tests.

Table 2

$$H_2N\underset{\underset{NH}{\|}}{C}NH(CH_2)_m\underset{R_1}{CH}CH_2CO-R_2-NH(CH_2)_4NH(CH_2)_3NH_2$$

| Comp.No. | m | $R_1$ | $R_2$ | (amino acid residue) | |
|---|---|---|---|---|---|
| 1 | 4 | H | $-NH-CH_2-CO-$ | (L) | (glycyl) |
| 2 | 4 | H | $CH_2OH$<br>\\<br>$-NH-CH-CO-$ | (L) | (L-seryl) |
| 3 | 4 | (S) OH | 〃 | 〃 | (L-seryl) |
| 4 | 4 | H | 〃 | (D) | (D-seryl) |
| 5 | 4 | H | $CH_3$<br>\\<br>$-NH-CH-CO-$ | (L) | (L-alanyl) |
| 6 | 4 | H | $-NH-CH_2CH_2-CO$ | | (β-alanyl) |
| 7 | 4 | H | $-NH-CH_2CH_2CH_2-CO-$ | (γ-aminobutanoyl) | |
| 8 | 4 | H | 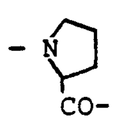 | (L) | (L-prolyl) |
| 9 | 4 | H | $CH_2CH(CH_3)_2$<br>\|<br>$-NH-CH-CO-$ | (L) | (L-leucyl) |

| Comp.No. | m | $R_1$ | $R_2$ | (amino acid residue) |
|---|---|---|---|---|
| 10 | 4 | H | 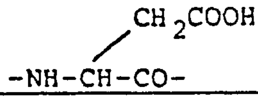 | (L) (aspartyl) |
| 11 | 4 | H | 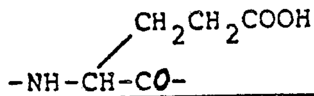 | (L) (L-glutamyl) |
| 12 | 4 | H | 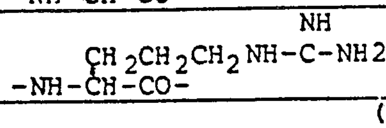 | (L) (L-arginyl) |
| 13 | 4 | H | 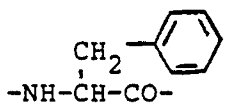 | (L) (L-phenylalanyl) |
| 14 | 4 | H | 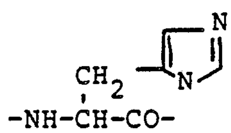 | (L) (L-histidyl) |
| 15 | 4 | H | 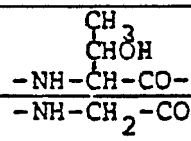 | (L) (L-threonyl) |
| 16 | 4 | (S)-$OCOCH_3$ | $-NH-CH_2-CO-$ | (glycyl) |
| 17 | 6 | H | 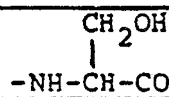 | (L) (L-seryl) |
| 18 | 4 | H | 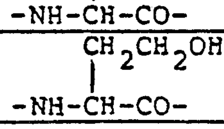 | (DL) (DL-homoseryl) |

1. Stability of the compounds of this invention in the form of aqueous solutions

(1) Testing method

Each of the compounds of this invention was dissolved in water to a concentration of 0.5 (w/w)%. The aquoeus solution was maintained at 40 ± 1°C, and sampled at certain time intervals. Each sample was subjected to high-performance liquid chromatographic analysis to measure the area of the peak of the compound at each sampling time, and to calculate the ratio of the area of the peak after the indicated period of time to that at the first sampling time. The results of the calculation were used to determine the contents (%) of the compound of this invention in the aqueous solution after the indicated periods of time, with the initial content of said compound set at 100%.

(2) Test results

Table 3 gives the results of the above test.

Table 3 Residual content (%) of the compound of this invention in aqueous solution

| Comp. No. \ Time that elapsed | 0 | 12 | 24 | 48 | 72 | 120 | 168 |
|---|---|---|---|---|---|---|---|
| 1 | 100 | 99.7 | 100 | 99.7 | 99.7 | 99.8 | 99.9 |
| 2 | " | 100 | 100 | 99.8 | 100 | 99.7 | 99.8 |
| 3 | " | 99.3 | 99.5 | 100 | 99.6 | 99.7 | 100 |
| 4 | " | 101 | 100 | 100 | 99.7 | 99.6 | 100 |
| 5 | " | 99.8 | 99.8 | 100 | 99.6 | 99.0 | 99.9 |
| 6 | " | 100 | 100 | 99.6 | 99.6 | 99.9 | 100 |
| 7 | " | 99.9 | 100 | 99.8 | 99.9 | 100 | 99.8 |
| 8 | " | 100 | 100 | 99.6 | 99.8 | 99.8 | 100 |
| 9 | " | 99.7 | 99.9 | 99.8 | 100 | 100 | 99.9 |
| 10 | " | 99.9 | 99.4 | 99.8 | 100 | 99.8 | 99.9 |
| 11 | " | 99.5 | 99.7 | 101 | 99.5 | 100 | 99.3 |
| 12 | " | 100 | 99.4 | 99.7 | 99.9 | 100 | 99.7 |
| 13 | " | 99.8 | 99.5 | 99.9 | 100 | 101 | 100 |
| 14 | " | 99.1 | 99.0 | 99.6 | 99.5 | 99.9 | 99.6 |
| 15 | " | 99.2 | 99.8 | 99.1 | 100.2 | 99.6 | 100.1 |
| 16 | " | 99.9 | 99.5 | 99.6 | 99.2 | 99.6 | 99.6 |
| 17 | " | 99.6 | 100.2 | 99.8 | 99.7 | 100 | 99.3 |
| 18 | " | 100 | 99.5 | 99.7 | 99.3 | 99.7 | 99.5 |
| Spergualin | " | 94.6 | 90.8 | 84.6 | 78.5 | 69.9 | 65.1 |

2. Growth-inhibiting action of the compounds of the invention on Bacillus subtilis

(1) Testing method

Each of the compounds of this invention was dissolved in nutrient agar media to final concentrations of 100, 50, 25, 12.5, 6.25, 3.13 and 1.56 µg/ml, to prepare agar plates for testing. Bacillus subtilis PCI219 was adjusted to a content of 1 × $10^6$ viable cells/ml, in a culture broth, and a loopful of the broth was applied onto the agar plates. The plates were incubated for 20 hours at 37°C undr stationary conditions, and observed for the formation of colonies of Bacillus subtilis. The lowest of the concentrations at which no colonies were formed was taken to be minimum inhibitory concentration (MIC).

(2) Test results

Table 4 shows the growth-inhibiting action of typical examples of the compounds of this invention on Bacillus subtilis. The intensity of this action is expressed by MIC.

Table 4 Growth-inhibiting action of the compounds of this invention on Bacillus subtilis

| Compound (Example No.) | MIC (µg/ml) |
|---|---|
| 1 | 6.25 |
| 2 | 12.5 |
| 3 | 6.25 |
| 4 | 12.5 |
| 5 | 25.0 |
| 6 | 25.0 |
| 7 | 25.0 |
| 8 | > 100 |
| 9 | > 100 |
| 10 | > 100 |
| 11 | > 100 |
| 12 | 12.5 |
| 13 | > 100 |
| 14 | 50.0 |
| 15 | 100 |
| 16 | 200 |
| 17 | 6.25 |
| 18 | 100 |

3. In vitro proliferation-inhibiting action of the compounds of this invention on mouse leukemia L1210 cells.

(1) Testing method

Leukemia L1210 cells ($1 \times 10^5$/0.2 ml) were transplanted to female DBA/2 mice intraperitoneally. Four days later, the ascitic fluid was collected and centrifuged to obtain proliferated L1210 cells. The L1210 cells collected were added to RPMI1640 medium containing bovine fetal serum and 2-mercaptoethanol, to obtain a suspension of L1210 cells having a final concentration of $5 \times 10^4$cells/0.9 ml. Each of the compounds of this invention was dissolved in said culture medium to prepare solutions with final concentrations ranging from 0.062 to 100 μg/ml. 0.9 ml of the L1210 cell suspension and 0.1 ml of each test solution were mixed in petri dishes, and incubated for 48 hours at 37°C in an incubator under an atmosphere of carbon dioxide gas. The number of L1210 cells was counted before and after the incubation, and that concentration of the compound which inhibited the proliferation of L1210 cells by 50% of the control's proliferation was determined ($IC_{50}$).

(2) Test results

Table 5 shows the proliferation-inhibiting action of typical examples of the compounds of this invention on mouse leukemia L1210 cells. The intensity of the action is expressed by the value of $IC_{50}$.

Table 5 In vitro proliferation-inhibiting action of compounds of this invention on mouse leukemia L1210 cells

| Compound (Example No.) | $IC_{50}$ (μg/ml) |
|---|---|
| 1 | 1.7 |
| 2 | 4.5 |
| 3 | 0.95 |
| 4 | 3.6 |
| 5 | 37 |
| 6 | 0.84 |
| 7 | 1.9 |
| 9 | 70 |
| 10 | 130 |
| 12 | 1.5 |
| 13 | 2.8 |
| 14 | 51 |
| 15 | 180 |
| 17 | 11.3 |
| 18 | 150 |
| Spergualin | 4.6 |

4. Life-prolonging effect and toxicity of the compounds of this invention in mouse leukemia L1210

(1) Testing method

Leukemia L1210 cells ($1 \times 10^5$/0.2 ml) were transplanted intraperitoneally into male CDF,—SLC mice (6 mice/group). Each of the compounds of this invention was diluted with physiological saline to various concentrations. Each dilution was administered at a dose of 0.1 ml/10 g of body weight once daily for 9 days, starting on the day after the day of the transplantation. The control group was invention.

The mice were observed for 60 days, beginning on the day after the day of L1210 transplantation, to examine how many days each mouse survived. The average number of days that the group receiving the compound of this invention survived was calculated. The figure obtained was divided by the average number of survival days for the control group, and the quotient was multiplied by 100 to give an increase of life span (T/C (%)). T/C of 125 or more was considered to show a good effect.

Change in body weight, the measure of the toxicity of the compound of this invention, was expressed by a difference between the change in body weight for the group receiving the compound of this invention and that for the control group receiving physiological saline and undergoing no transplantation.

(2) Test results

Table 6 shows the life-prolonging effect of typical examples of the compounds of this invention on mouse leukemia L1210, and their toxicity. The life-prolonging effect is expressed by T/C, and the toxicity by the change in body weight.

Table 6 Life-prolonging effect of the compounds of this invention on mouse leukemia L1210, and their toxicity

| Compound (Example No.) | Dose (mg/kg/day) | T/C (%) | Change in body weight (g) |
|---|---|---|---|
| Control | 0.00 | 100 | 1.8 |
| 1 | 50.00 | 12.9 | – |
| | 25.00 | > 279 | -1.6 |
| | 12.50 | > 528 | +0.2 |
| | 6.25 | > 769 | +0.9 |
| | 3.13 | > 667 | +0.8 |
| | 1.56 | > 388 | +1.0 |
| | 0.78 | > 250 | +2.7 |
| | 0.39 | 126 | +1.8 |
| 2 | 50.00 | 12.8 | – |
| | 25.00 | 100 | – |
| | 12.50 | > 612 | -0.2 |
| | 6.25 | > 705 | +1.0 |
| | 3.13 | >769 | +0.6 |
| | 1.56 | > 769 | +1.3 |
| | 0.78 | > 346 | +1.5 |
| | 0.39 | 124 | +2.7 |
| Spergualin | 50.00 | > 342 | +0.2 |
| | 25.00 | > 524 | +1.1 |
| | 12.50 | > 700 | +0.8 |
| | 6.25 | > 769 | +0.9 |
| | 3.13 | > 665 | +1.2 |
| | 1.56 | > 224 | +2.5 |
| | 0.78 | 129 | +0.8 |

As clear from the above experimental examples, the compounds of this invention have good biological activities, high stability, and are promising as pharmaceuticals such as antitumor agents. Of these compounds expressed by the general formula (I), those in which m stands for 4 to 6, $R_1$ represents a hydrogen atom or a hydroxyl group, and the amino acid residue as $R_2$ is glycyl, seryl, β-alanyl, γ-aminobutanoyl, arginyl or phenylalanyl are preferred because of their better activities.

The present invention will be described in more detail by reference to Examples to follow. In the Examples, Rf value in thin-layer chromatography (TLC) was determined by applying a solution of the desired product onto a silica gel 60 $F_{254}$ plate (0.25 mm thick, a product of Merck) at a certain position (so-called origin), developing this plate with the indicated developer over a distance of about 8 cm, and dividing the distance from the origin to the center of the appearing spot for the desired product by the distance from the origin to the forward end of the development zone (so-called solvent front). The desired product was detected by using UV (2537 Å), ninhydrin and Sakaguchi's reagent.

Example 1

10-[N-(7-guanidinoheptanoyl)glycyl]-1,5,10-triazadecane trihydrochloride

20.8 grams (about 24 mmols) of 10-[N-(7-guanidinoheptanoyl)glycyl]-1,5-dibenzyloxycarbonyl-1,5,10-triazadecane hydrochloride, an oily substance, was dissolved in a mixture of 300 ml of methanol and 10 ml of acetic acid. 0.50 g of palladium black was added to the solution, and the mixture was catalytically reduced for 3 hours at room temperature and atmospheric pressure. After the reaction, the catalyst was removed by filtration, and the filtrate was concentrated under reduced pressure to obtain 19.5 g of oily matter. The oily matter was dissolved in 70 ml of distilled water, and the solution was passed through a 1500 ml column of CM-Sephadex® C—25 ($Na^+$). Gradient elution was made between 7500 ml of distilled water and 7500 ml of an aqueous solution of 1M sodium chloride.

Active fractions were collected, and concentrated to dryness under reduced pressure. Methanol was added to the residue, and the insoluble sodium chloride was removed by filtration. This purification step was carried out once more. To remove a small amount of sodium chloride that remained unremoved, the resulting oily matter was dissolved in 50 ml of methanol, and the solution was passed through a column (400 ml) of Sephadex® LH—20. The column was eluted with methanol, and active fractions were collected, followed by concentrating them under reduced pressure to obtain 5.30 g of an oily substance. The oily substance was dissolved in 20 ml of distilled water, and the insolubles were removed by filtration. The filtrate was freeze-dried to obtain 5.20 g of the desired product (yield: 45.1%).

mp: 163—165°C

NMR ($DMSO-d_6$): = 0.9—1.8 (b, 12H), 1.8—2.4 (b, 4H), 2.6—3.3 (b, 10H), 3.63 (d, 2H, J=5Hz), 6.9—9.2 (b, 12H)

IR (KBr): 3410, 3310, 3150, 2930, 1640, 1520, 1470, 1410, 1160, 965

TLC (n-propanol:pyridine:water:acetic acid — 6:4:3:2 v/v) Rf=0.4

MS (FD): m/z 372 (M+1)

## Example 2

10-[N-(7-guanidinoheptanoyl)-L-seryl]-1,5,10-triazadecane trihydrochloride

2.50 g (3.14 mmols) of 10-[N-(7-guanidinoheptanoyl)-O-benzyl-L-seryl]-1,5-dibenzyloxycarbonyl-1,5,10-triazadecane hydrochloride was dissolved in a mixture of 30 ml of methanol and 1 ml of acetic acid. 0.1 g of palladium black was added to the solution, and the mixture was catalytically reduced for 5 hours at atmospheric pressure with heating at 50°C. After the reaction, the catalyst was removed by filtration, and the filtrate was concentrated under reduced pressure to obtain 1.7 g of oily matter.

The oily matter was dissolved in 6 ml of distilled water, and the solution was chromatographed on a 300 ml column of CM-Sephadex® C—25 ($Na^+$). The column was eluted by gradient elution between 200 ml of distilled water and 200 ml of an aqueous solution of 1.5M sodium chloride.

Active fractions were collected, and dried under reduced pressure. Methanol was added to the residue, and the insoluble sodium chloride was removed by filtration. This purification step was performed again. To remove a small amount of sodium chloride that remained unremoved, the resulting oily material was dissolved in 5 ml of methanol, and the solution was passed through a 100 ml column of Sephadex® LH—20. The column was eluted with methanol, active fractions were collected, and the collected fractions were concentrated under reduced pressure. The resulting oily matter was dissolved in 5 ml of distilled water, and the insolubles were removed by filtration. The residue was freeze-dried to obtain 0.43 g of the desired product in yield of 45.1%.

NMR ($DMSO-d_6$): = 0.8—1.8 (b, 12H), 1.8—2.4 (b, 4H), 2.5—3.4 (b, 10H), 3.57 (d, 2H, J=5Hz), 4.18 (m, 1H), 5.5—6.5 (b, 1H), 6.7—9.5 (b, 12H)

IR (KBr): ($cm^{-1}$) = 3350, 2940, 1640, 1535, 1465, 1375, 1160, 1060, 965

TLC (n-propanol:pyridine:acetic acid:water = 6:4:3:2 v/v)

$[\alpha]_D^{27}$: −15.2° (c = 1.0, $H_2O$)

MS (FD): m/z 402 (M+1)

The compounds of the general formula (V) shown in the following table were treated in accordance with the procedure of Example 1 or 2 to obtain the compounds of the general formula (I) tabulated below.

| Example | Compound of formula (II) | Compound of formula (I) |
|---|---|---|
| 3 | 10-[N-((S)-7-guanidino-3-hydroxyheptanoyl)-O-benzyl--L-seryl]-1,5-dibenzyloxycarbonyl-1,5,10-triazadecane hydrochloride<br><br>NMR (DMSO-$d_6$)<br>$\delta$=0.9 - 2.0 (b, 12H)<br>2.1 - 2.4 (d, 2H, J=6Hz)<br>2.6 - 3.5 (b, 10H)<br>3.60 (d, 2H, J=5Hz)<br>3.8 - 4.7 (b, 2H)<br>4.48 (s, 2H)<br>4.5 - 5.5 (b, 1H)<br>5.01 (s, 2H)<br>5.05 (s, 2H)<br>6.7 - 8.3 (b, 8H)<br>7.27 (s, 5H)<br>7.30 (s, 10H)<br>TLC (n-butanol:water:<br>acetic acid = 4:1:1 v/v)<br>Rf = 0.6 | 10-[N-((S)-7-guanidino-3-hydroxyhoptanoyl)-L-seryl] 1,5,10-triazadecane trihydrochloride<br><br>NMR (DMSO-$d_6$)<br>$\delta$=1.0 - 1.8 (b, 10H)<br>1.8 - 2.4 (b, 4H)<br>2.6 - 3.5 (b, 10H)<br>3.60 (d, 2H, J=5Hz)<br>3.7 - 4,3 (b, 2H)<br>4.3 -5.5 (b, 2H)<br>6.9 - 9.1 (b, 12H)<br>IR (KBr)<br>($cm^{-1}$) = 3365, 2945, 1650<br>1540, 1460, 1170<br>1060, 965<br><br>TLC (n-butanol:pyridine:<br>water:acetic acid<br>= 6:4:3:2 v/v)<br>Rf = 0.3<br>$[\alpha]_D^{23}$ -14.7° (c = 1.0 $H_2O$)<br>MS (FD) m/z 418 (M + 1) |

| Example | Compound of formula (II) | Compound of formula (I) |
|---|---|---|
| 4 | 10-[N-(7-guanidinoheptanoyl)-O-benzyl-D-seryl]-1,5-dibenzyloxycarbonyl-1,5,10-triazadecane hydrochloride<br><br>NMR (DMSO-$d_6$)<br>$\delta$ =0.9 - 2.0 (b, 14H)<br>2.0 - 2.4 (d, 2H)<br>2.7 - 3.5 (b, 10H)<br>3.58 (d, 2H, J=5Hz)<br>4.2 - 4.8 (b, 1H)<br>4.47 (s, 2H)<br>4.5 - 5.5 (b, 1H)<br>5.01 (s, 2H)<br>5.04 (s, 2H)<br>6.7 - 8.3 (b, 8H)<br>7.27 (s, 5H)<br>7.30 (s, 10H)<br>TLC (chloroform:methanol: acetic acid = 9:1:0.5 v/v)<br>Rf = 0.2 | 10-[N-(7-guanidinoheptanoyl)-D-seryl]-1,5,10-triazadecane trihydrochloride<br><br>NMR (DMSO-$d_6$)<br>$\delta$ =0.8 - 1.9 (b, 12H)<br>1.8 - 2.4 (b, 4H)<br>2.6 - 3.4 (b, 10H)<br>3.58 (d, 2H, J=5Hz)<br>4.18 (m, 1H)<br>5.0 - 5.8 (b, 1H)<br>6.7 - 9.4 (b, 12H)<br>IR (KBr)<br>($cm^{-1}$) = 3350, 2940, 1640<br>1535, 1460, 1360<br>1160, 1060,<br><br>TLC (n-butanol:pyridine: water:acetic acid = 6:4:3:2 v/v)<br>Rf = 0.4<br>$[\alpha]_D^{26}$ +15.3° (c = 1.0 $H_2O$)<br>MS (FD) m/z 402 (M + 1) |

| Example | Compound of formula (II) | Compound of formula (I) |
|---|---|---|
| 5 | 10-[N-(7-guanidinoheptanoyl)-L-alanyl]-1,5-dibenzyloxycarbonyl-1,5,10-triazadecane hydrochloride<br><br>NMR (DMSO-$d_6$)<br>$\delta$=0.9 - 2.0 (b, 17H)<br>2.0 - 2.3 (d, 2H)<br>2.3 - 3.5 (b, 10H)<br>4.0 - 4.5 (b,1H)<br>4.99 (s, 2H)<br>5.03 (s, 2H)<br>6.3 - 8.7 (b, 8H)<br>7.3 (s, 10H)<br><br>TLC (n-propanol:pyridine:water:acetic acid = 6:4:3:2 v/v)<br>Rf = 0.8 | 10-[N-(7-guanidinoheptanoyl-L-alanyl]-1,5,10-triazadecane trihydrochloride<br><br>NMR (DMSO-$d_6$)<br>$\delta$=0.9 - 1.8 (b, 15H)<br>1.8 - 2.4 (b, 4H)<br>2.6 - 3.4 (b, 10H)<br>3.9 - 4.5 (b, 1H)<br>5.9 - 8.2 (b, 12H)<br><br>IR (KBr)<br>($cm^{-1}$) = 3380, 2940, 1640, 1540, 1370, 1160, 965<br>TLC (n-propanol:pyridine:water:acetic acid = 6:4:3:2 v/v)<br>Rf = 0.4<br>$[\alpha]_D^{27}$ -21.6° (c = 1.2 $H_2O$)<br>MS (FD) m/z 386 (M + 1) |

| Example | Compound of formula (II) | Compound of formula (I) |
|---|---|---|
| 6 | 10-[N-(7-guanidinoheptanoyl)-β-alanyl]-1,5-dibenzyloxycarbonyl-1,5,10-triazadecane hydrochloride<br><br>NMR (DMSO-$d_6$)<br>δ=1.0 - 2.1 (b, 14H)<br>2.0 - 2.5 (d, 4H)<br>2.6 - 3.1 (b, 12H)<br>4.99 (s, 2H)<br>5.03 (s, 2H)<br>6.7 - 8.3 (b, 8H)<br>7.3 (s, 10H)<br><br>TLC (chloroform:methanol: 29% ammonia water = 2:2:1 v/v)<br>Rf = 0.8 | 10-[N-(7-guanidinoheptanoyl-β-alanyl]-1,5,10-triazadecane trihydrochloride<br><br>NMR (DMSO-$d_6$)<br>δ=1.0 - 1.8 (b, 12H)<br>1.9 - 2.5 (b, 6H)<br>2.6 - 3.5 (b, 12H)<br>7.0 - 9.3 (b, 12H)<br><br>IR (KBr)<br>($cm^{-1}$) = 3350, 2935, 1635<br>1545, 1460, 1365<br>1165, 965<br><br>TLC (chloroform:methanol: 29% ammonia water = 2:2:1 v/v)<br>Rf = 0.6<br>MS (FD) m/z 386 (M + 1) |

| Example | Compound of formula (II) | Compound of formula (I) |
|---|---|---|
| 7 | 10-[N-(7-guanidinoheptanoyl)-γ-aminobutanoyl-1,5-dibenzyloxycarbonyl-1,5,10-triazadecane hydrochloride<br><br>NMR (DMSO-$d_6$)<br>$\delta$=0.9 - 2.0 (b, 16H)<br>2.0 - 2.5 (d, 4H)<br>2.7 - 3.7 (b, 12H)<br>4.99 (s, 2H)<br>5.03 (s, 2H)<br>6.5 - 8.5 (b, 8H)<br>7.30 (s, 10H)<br><br>TLC (n-butanol:water:acetic acid = 4:1:1 v/v)<br>Rf = 0.6 | 10-[N-(7-guanidinoheptanoyl-γ-aminobutanoyl]-1,5,10-triazadecane trihydrochloride<br><br>NMR (DMSO-$d_6$)<br>$\delta$=0.9 - 1.8 (b, 14H)<br>1.8 - 2.4 (b, 6H)<br>2.6 - 3.4 (b, 12H)<br>6.7 - 9.2 (b, 12H)<br><br>IR (KBr)<br>($cm^{-1}$) = 3350, 2940, 1640<br>1550, 1460, 1360<br>TLC (n-propanol:pyridine:water:acetic acid = 6:4:3:2 v/v)<br>Rf = 0.4<br>MS (FD) m/z 400 (M + 1) |

| Example | Compound of formula (II) | Compound of formula (I) |
|---|---|---|
| 8 | 10-[N-(7-guanidinoheptanoyl-L-propyl]-1,5-dibenzyloxycarbonyl-1,5,10-tirazadecane hydrochloride<br><br>NMR (DMSO-$d_6$)<br>$\delta$=0.9 - 2.4 (b, 20H)<br>2.7 - 3.7 (d, 12H)<br>4.0 - 4.4 (b, 1H)<br>5.00 (s, 2H)<br>5.04 (s, 2H)<br>6.5 - 7.9 (b, 7H)<br>7.33 (s, 10H)<br><br>TLC (n-butanol:acetic acid: water = 4:1:1 v/v)<br>Rf = 0.3 | 10-[N-(7-guanidinoheptanoyl)-L-propyl]-1,5,10-triazadecane trihydrochloride<br><br>NMR (DMSO-$d_6$)<br>$\delta$ =1.0 - 2.4 (b, 20H)<br>2.5 - 3.9 (b, 12H)<br>4.0 - 4.5 (b, 1H)<br>6.7 - 9.3 (b, 11H)<br><br>IR (KBr)<br>($cm^{-1}$) = 3400, 2940, 1640<br>1450, 1160, 965<br>TLC (n-propanol:pyridine: water:acetic acid = 6:4:3:2 v/v)<br>Rf = 0.3<br>$[\alpha]_D^{27}$ -42.3$^o$ (c = 1.3, $H_2O$)<br>MS (FD) m/z 412 (M + 1) |

| Example | Compound of formula (II) | Compound of formula (I) |
|---|---|---|
| 9 | 10-[N-(7-guanidinoheptanoyl-L-leucyl]-1,5-dibenzyloxycarbonyl-1,5,10-triazadecane hydrochloride<br><br>NMR (DMSO-$d_6$)<br>$\delta$=0.6 - 1.0 (b, 6H)<br>1.1 - 1.9 (d, 16H)<br>1.9 - 2.4 (b, 3H)<br>2.7 - 3.5 (b, 10H)<br>3.8 - 4.6 (m, 1H)<br>5.06 (s, 2H)<br>5.10 (s, 2H)<br>6.8 - 8.3 (b, 8H)<br>7.33 (s, 10H)<br><br>TLC (n-butanol:acetic acid: water = 4:1:1 v/v)<br>Rf = 0.5 | 10-[N-(7-guanidinoheptanoyl-L-leucyl]-1,5,10-triazadecane trihydrochloride<br><br>NMR (DMSO-$d_6$)<br>$\delta$ =0.5 - 1.0 (b, 6H)<br>1.0 - 1.9 (b, 15H)<br>1.9 - 2.3 (b, 4H)<br>2.6 - 3.5 (b, 10H)<br>3.9 - 4.5 (b, 1H)<br>6.8 - 9.1 (b, 12H)<br><br>IR (KBr)<br>($cm^{-1}$) = 3400, 2945, 1645<br>1535, 1465, 1370<br>1165, 970<br>TLC (n-propanol:pyridine: water:acetic acid = 6:4:3:2 v/v)<br>Rf = 0.4<br>$[\alpha]_D^{27}$ -20.5° (c = 1.4, $H_2O$)<br>MS (FD) m/z 428 (M + 1) |

| Example | Compound of formula (II) | Compound of formula (I) |
|---|---|---|
| 10 | 10-[N-(-7-guanidinoheptanoyl)-β-benzyl-L-aspartyl]-1,5-dibenzyloxycarbonyl-1,5,10-triazadecane hydrochloride<br><br>NMR ($DMSO-d_6$)<br>$\delta$=1.0 - 2.5 (b, 14H)<br>1.9 - 2.4 (d, 2H)<br>2.4 - 3.7 (b, 12H)<br>4.3 - 4.9 (b, 1H)<br>5.00 (s, 2H)<br>5,05 (s, 4H)<br>5.2 - 6.4 (b, 3H)<br>6.7 - 8.0 (b, 5H)<br>7.33 (s, 15H)<br><br>TLC (n-butanol:water:acetic acid = 4:1:1 v/v)<br>Rf = 0.4 | 10-[N-(7-guanidinoheptanoyl)-L-aspartyl]-1,5,10-triazadecane trihydrochloride<br><br>NMR ($DMSO-d_6$)<br>$\delta$=0.9 - 1.8 (b, 12H)<br>1.8 - 2.6 (b, 6H)<br>2.7 - 3.5 (b, 10H)<br>4.1 - 4.7 (b, 1H)<br>6.9 - 8.7 (b, 13H)<br><br>IR (KBr)<br>($cm^{-1}$) = 3320, 2935, 1640<br>1550, 1470, 1390<br>1310, 1170, 965<br><br>TLC (n-butanol:pyridine:water:acetic acid = 6:4:3:2 v/v)<br>Rf = 0.4<br>$[\alpha]_D^{27}$ -10.3° (c = 1.5 $H_2O$)<br>MS (FD) m/z 430 (M + 1) |

| Example | Compound of formula (II) | Compound of formula (I) |
| --- | --- | --- |
| 11 | 10-[N-(-7-guanidinoheptanoyl)-L-glutaminyl]-1,5-dibenzyloxycarbonyl-1,5,10-triazadecane hydrochloride<br><br>NMR (DMSO-$d_6$)<br>$\delta$=0.9 - 2.4 (b, 20H)<br>2.6 - 3.8 (d, 10H)<br>3.9 - 4.3 (b, 1H)<br>4.99 (s, 2H)<br>5.04 (s, 2H)<br>6.5 - 8.3 (b, 10H)<br>7.34 (s, 10H)<br><br>TLC (chloroform:methanol:acetic acid = 8:2:0.5 v/v)<br>Rf = 0.1 | 10-[N-(7-guanidinoheptanoyl)-L-glutaminyl]-1,5,10 triazadecane trihydrochloride<br><br>NMR (DMSO-$d_6$)<br>$\delta$=0.8 - 1.8 (b, 14H)<br>1.8 - 2.4 (b, 6H)<br>2.6 - 3.8 (b, 12H)<br>4.0 - 4.3 (m, 1H)<br>6.5 - 9.2 (b, 12H)<br><br>IR (KBr)<br>($cm^{-1}$) = 3400, 2940, 1655<br>1540, 1455, 1160<br>965<br><br>TLC (n-propanol:pyridine:water:acetic acid = 6:4:3:2 v/v)<br>Rf = 0.4<br>$[\alpha]_D^{23}$ -11.2° (c = 1.1 $H_2O$)<br>MS (FD) m/z 443 (M + 1) |

| Example | Compound of formula (II) | Compound of formula (I) |
|---|---|---|
| 12 | 10-[N -(-7-guanidinoheptanoyl)-$N^G$-nitro-L-arginyl]-1,5-dibenzyloxycarbonyl-1,5,10- triazadecane hydrochloride<br><br>NMR ($DMSO-d_6$)<br>$\delta$= 1.0-1.9 (b, 18H), 1.9-2.5(b, 2H), 2.7-3.8(b, 12H), 4.1-4.5(b, 1H), 5.01(a, 2H), 5.04(a, 2H), 6.0-8.4(b, 11H), 7.30(a, 10H)<br>TLC(n-propanol:pyridine:water:acetic acid = 6:4:3:2 v/v)<br><br>Rf = 0.8 | 10-[N-(7-guanidinoheptanoyl)-L-arginyl]-1,5,10-triazadecane trihydrochloride<br><br>NMR ($DMSO-d_6$)<br>$\delta$= 1.0-1.9(b, 16H), 1.9-2.3(b, 4H), 2.7-3.8(b, 12H), 4.0-4.5(b, 1H), 6.5-9.2(b, 17H).<br>IR(KBr)<br>($cm^{-1}$)=3330, 2930, 1640, 1530, 1460, 1365, 1250, 1160, 1100.<br>TLC(n-propanol:pyridine:water:acetic acid = 6:4:3:2 v/v)<br>Rf = 0.2<br>$[\alpha]_D^{23}$-7.2°(c=1.2, $H_2O$)<br>MS(FD)<br>m/z 471(M+1). |

| Example | Compound of formula (II) | Compound of formula (I) |
|---|---|---|
| 13 | 10-[N-(-7-guanidinoheptanoyl)-L-phenylalanyl]-1,5-dibenzyloxycarbonyl-1,5,10-triazadecane hydrochloride<br><br>NMR (DMSO-$d_6$)<br>$\delta$= 0.9-2.4 (b, 16H), 2.7-3.7(b,10H), 3.5(s, 2H), 4.3-4.7(b, 1H), 4.98(a, 2H), 5.02(s, 2H), 6.9-8.3(b, 8H), 7.17(s, 5H), 7.30(s, 10H).<br>TLC(n-butanol:water:acetic acid = 4:1:1 v/v)<br><br>Rf = 0.5 | 10-[N-(7-guanidinoheptanoyl)-L-phenylalanyl]-1,5,10-triazadecane trihydrochloride<br><br>NMR (DMSO-$d_6$)<br>$\delta$= 0.9-1.8(b, 12H), 1.9-2.3(b, 4H), 2.7-3.8(b, 12H), 4.2-4.7(b, 1H), 6.9-9.3(b, 12H), 7.22 (s. 5H).<br>IR(KBr)<br>($cm^{-1}$)=3320, 2930, 1645 1530, 1455, 1370, 965, 700.<br>TLC(n-propanol:pyridine: water:acetic acid = 6:4:3:2 v/v)<br>Rf = 0.4<br>$[\alpha]_D^{27}$-6.7°(c=1.2, $H_2O$)<br>MS(FD)<br>m/z 462(M+1). |

| Example | Compound of formula (II) | Compound of formula (I) |
|---|---|---|
| 14 | 10-[N-(-7-guanidinohepta-<br>noyl)-$N^{im}$-benzyloxycarbonyl-<br>L-histidyl]-1,5-dibenzyloxy-<br>carbonyl-1,5,10-triazadocane<br>hydrochloride<br><br>NMR (DMSO-$d_6$)<br>$\delta$=0.9 - 2.0 (b, 14H)<br>2.0 - 3.5 (d, 14H)<br>4.0 - 4.5 (b, 1H)<br>4.99 (s, 2H)<br>5.03 (s, 2H)<br>5.10 (s, 2H)<br>6.7 - 8.0 (b, 2H)<br>7.31 (s, 15H)<br><br>TLC (n-butanol:water:<br>acetic acid = 4:1:1 v/v)<br>Rf = 0.4 | 10-[N-(7-guanidinohepta-<br>noyl)-L-Histidyl]-1,5,10-<br>triazadecane<br>trihydrochloride<br><br>NMR (DMSO-$d_6$)<br>$\delta$=0.9 - 1.8 (b, 12H)<br>1.8 - 2.3 (b, 4H)<br>2.7 - 3.5 (b, 12H)<br>4.3 - 4.9 (b, 1H)<br>6.3 - 9.0 (b, 17H)<br><br>IR (KBr)<br>($cm^{-1}$) = 3370, 2940, 1650<br>1540, 1460, 1370<br>1165, 1080<br>TLC (n-propanol:pyridine:<br>water:acetic acid<br>= 6:4:3:2 v/v)<br>Rf = 0.3<br>$[\alpha]_D^{27}$ -2.8° (c = 1.2 $H_2O$)<br>MS (FD) m/z 452 (M + 1) |

| Example | Compound of formula (II) | Compound of formula (I) |
|---|---|---|
| 15 | 10-[N-(-7-guanidinoheptanoyl)-O-benzyl-L-threonyl]-1,5-dibenzyloxycarbonyl-1,5,10-triazadecane hydrochloride<br><br>NMR (DMSO-$d_6$)<br>$\delta$=0.6 - 2.4 (b, 19H)<br>2.6 - 3.5 (b, 10H)<br>3.5 - 4.3 (b, 2H)<br>4.3 - 4.6 (b, 2H)<br>4.6 - 5.2 (b, 3H)<br>4.98 (s, 2H)<br>5.02 (s, 2H)<br>6.5 - 8.0 (b, 5H)<br>7.2 (s, 5H)<br>7.3 (s, 10H)<br><br>TLC (n-butanol:water:acetic acid = 4:1:1 v/v)<br>Rf = 0.7 | 10-[N-(7-guanidinoheptanoyl)-L-threonyl]-1,5,10-triazadecane trihydrochloride<br><br>NMR (DMSO-$d_6$)<br>$\delta$=1.05 (d, 3H, J=6Hz)<br>0.8 - 1.9 (b, 12H)<br>1.9 - 2.4 (b, 4H)<br>2.6 - 4.3 (b, 15H)<br>6.5 - 9.5 (b, 10H)<br><br>IR (KBr)<br>($cm^{-1}$) = 3330, 2940, 1650<br>1530, 1465, 1380<br>1160, 1110, 930<br>TLC (n-butanol:pyridine:water:acetic acid = 6:4:3:2 v/v)<br>Rf = 0.2<br>$[\alpha]_D^{22}$ -13.1° (c = 1.1 $H_2O$)<br>MS (FD) m/z 416 (M + 1) |

| Example | Compound of formula (II) | Compound of formula (I) |
|---|---|---|
| 16 | 10-[N-((S)-7-guanidino-3-acetoxyheptanoyl)-glycyl]-1,5-dibenzloxycarbonyl-1,5,10-triazadecane hydrochloride<br><br>NMR (DMSO-$d_6$)<br>$\delta$=0.9 - 1.9 (b, 12H)<br>2.0 (s, 3H)<br>2.2 - 2.7 (b, 2H)<br>2.7 - 3.8 (b, 12H)<br>4.9 - 5.3 (b, 1H)<br>4.98<br>5.02 (s,s 4H)<br>5.7 - 9.0 (b, 8H)<br>7.3 (s, 10H)<br><br>TLC (n-butanol:water:acetic acid = 4:1:1 v/v)<br>Rf = 0.8 | 10-[N-((S)-7-guanidino-3-acetoxyheptanoyl)-glycyl-1,5,10-triazadecane trihydrochloride<br><br>NMR (DMSO-$d_6$)<br>$\delta$=1.0 - 1.8 (b, 10H)<br>1.8 - 2.2 (b, 2H)<br>2.0 (s, 3H)<br>2.2 - 2.7 (b, 2H)<br>2.7 - 3.5 (b, 10H)<br>3.65 (b, 2H, J=5Hz)<br>4.9 - 5.3 (b, 1H)<br>5.7 - 9.6 (b, 12H)<br><br>IR (KBr)<br>($cm^{-1}$) = 3390, 2950, 1720<br>1650, 1545, 1455<br>1380, 1250, 1170<br>1030<br><br>TLC (n-propanol:pyridine:water:acetic acid = 6:4:3:2 v/v)<br>Rf = 0.27<br>$[\alpha]_D^{20.6}$ -1.4° (c = 1.14, $H_2O$)<br>MS (FD) m/z 417 (M + 1) |

| Example | Compound of formula (II) | Compound of formula (I) |
|---|---|---|
| 17 | 10-[N-(9-guanidinohepta-noyl)-O-benzyl-L-seryl]-1,5-benzyloxycarbonyl-1,5,10-triazadecane hydrochloride<br><br>NMR (DMSO-$d_6$)<br>$\delta$=1.0 - 2.0 (b, 18H)<br>2.0 - 2.4 (b, 2H)<br>2.7 - 3.5 (b, 10H)<br>3.58 (d, 2H, J=6Hz)<br>4.2 - 4.8 (b, 1H)<br>4.47 (s, 2H)<br>5.01 (s, 2H)<br>5.05 (s, 2H)<br>6.9 - 8.3 (b, 8H)<br>7.29 (s, 5H)<br>7.33 (s, 10H)<br><br>TLC (chloroform:methanol: acetic acid = 9:1:0.3 v/v)<br>Rf = 0.2 | 10-[N-(9-guanidinohepta-noyl)-L-seryl]-1,5,10-triazadecane trihydrochloride<br><br>NMR (DMSO-$d_6$)<br>$\delta$=1.0 - 1.8 (b, 16H)<br>1.8 - 2.4 (b, 4H)<br>2.6 - 3.5 (b, 11H)<br>3.55 (d, 2H, J=5Hz)<br>4.0 - 4.3 (b, 1H)<br>7.0 - 9.6 (b, 12H)<br><br>IR (KBr)<br>($cm^{-1}$) = 3350, 2940, 1655 1540, 1470, 1160 1060<br>TLC (n-propanol:pyridine water:acetic acid = 6:4:3:2 v/v)<br>Rf = 0.6<br>$[\alpha]_D^{25}$ -5.0° (c = 0.5 $H_2O$)<br>MS (FD) m/z 430 (M + 1) |

Example 18

1.04 g (about 1.5 mmols) of 10-[N-(7-guanidinoheptanoyl)-DL-homoseryl]-1,5-di-tert.-butoxycarbonyl-1,5,10-triazedecane acetate was dissolved, while being cooled, in 3 ml of trifluoroacetic acid. Then, the solution was stirred for 5 hours at room temperature to carry out the elimination of the tert.-butoxycarbonyl group.

After the reaction, the trifluoroacetic acid was distilled off by concentration under reduced pressure. To the residue was added 10 ml of 1N hydrochloric acid, and the mixture was concentrated under reduced pressure to obtain 0.97 g of oily matter. The oily matter was dissolved in 10 ml of distilled water, and the solution was chromatographed on a 230 ml column of CM-Sephadex® C—25 ($Na^+$). The column was eluted using a gradient elution between 1200 ml of distilled water and 1200 ml of an aqueous solution of 0.9M sodium chloride.

Active fractions were collected, and dried under reduced pressure. Methanol was added to the dried material, and the insoluble sodium chloride was removed by filtration. This purification step was performed again. To remove a small amount of sodium chloride that remained unremoved, the resulting oily matter was dissolved in 5 ml of methanol, and passed through a 100 ml column of Sephadex® LH—20. The column was eluted with methanol, and active fractions were collected and concentrated under reduced pressure to obtain 0.38 g of an oily substance. This oily substance was dissolved in 4 ml of distilled water,

and the insolubles were removed by filtration. The filtrate was freeze-dried to obtain 0.37 g of the desired product in yield of 45%.

NMR (DMSO-$d_6$): = 0.6—2.0 (b, 14H), 2.0—2.3 (b, 4H), 2.6—4.0 (b, 15H), 4.0—4.7 (b, 1H), 6.0—9.5 (b, 10H).

IR (KBr): ν ($cm^{-1}$) = 3380, 2940, 1650, 1530, 1470, 1380, 1165, 1055, 965.

TLC (n-propanol:pyridine:water:acetic acid = 6:4:3:2 v/v): Rf = 0.4.,

MS (FD): m/z 416 (M+1).

Referential Example 1

(1) 10-(N,N-phthalylglycyl)-1,5-dibenzyloxycarbonyl-1,5,10-triazadecane

1.24 g (30.0 mmols) of 1,5-dibenzyloxycarbonyl-1,5,10-triazadecane was dissolved in 100 ml of tgetrahydrofuran, and 4.90 ml (35.0 mmols) of triethylamine was added to the solution under cooling with ice, 10.6 g (35.0 mmols) of an ester of phthalylglycine with N-hydroxysuccinimide was further added to the mixture, and the system was reacted overnight at room temperature.

The reaction mixture was dried under reduced pressure, and the residue was dissolved in 1200 ml of ethyl acetate. The ethyl acetate solution was washed with a 5% sodium hydrogencarbonate, 0.5N hydrochloric acid, and a saturated aqueous solution of sodium chloride in this order. The ethyl acetate layer was dried over anhydrous sodium sulfate, and then, the desiccant was removed by filtration. The filtrate was concentrated under reduced pressure, and ethyl acetate and ethyl ether were added to the residue to crystallize it. The crystals formed were collected by filtration, and dried to obtain 14.6 g of the desired product in a yield of 81.0%.

m.p. 102—104°C

NMR (DMSO-$d_6$): δ = 1.0—2.2 (b, 6H), 2.7—3.6 (b, 8H), 4.20 (s, 2H), 5.01 (s, 2H), 5.03 (s, 2H), 6.8—8.4 (b, 2H), 7.30 (s, 10H), 7.84 (s, 4H).

TLC (chloroform:methanol:acetic acid = 95:5:3 v/v): Rf = 0.4.

The starting material, 1,5-dibenzyloxycarbonyl-1,5,10-triazadecane, was synthesized in the following manner. 55.0 g (350 mmols) of 1-(4-aminobutyl)hexahydro-pyrimidine and 92.0 g (420 mmols) of ethoxycarbonylphthalimide were dissolved in 580 ml of dimethyl sulfoxide. To the solution was added 42.0 g (700 mmols) of glacial acetic acid, and the mixture was reacted overnight at room temperature with stirring, the reaction mixture was concentrated under reduced pressure by means of a vacuum pump.

The residue was dissolved in 200 ml of distilled water, and the solution was adjusted to a pH of 1.0 by addition of concentrated hydrochloric acid, followed by concentrating the solution under reduced pressure. The residue was recrystallized from ethanol to obtain 46.9 g of 10-phthalyl-1,5,10-triazedecane dihydrochloride as a pale yellow substance in a yield of 38.5%.

m.p. 244—246°C.

NMR ($D_2O$): δ = 1.5—2.0 (b, 4H), 2.0—2.5 (m, 2H), 2.9—3.5 (b, 6H), 3.5—3.9 (b, 2H), 7.76 (s, 4H).

27.9 g (80.0 mmols) of the resulting 10-phthalyl-1,5,10-triazedecane dihydrochloride was dissolved in 300 ml of chloroform. To the solution were added 43.9 g (160 mmols) of benzyl-S-4,6-dimethylpyrimidin-2-yl-thiolcarbonate and 17.8 g (17.6 mmols) of triethylamine, and the mixture was reacted for 6 hours at room temperature with stirring. The reaction mixture was washed with 1N hydrochloric acid and an aqueous solution of sodium chloride in this order, and dried over anhydrous magnesium sulfate. The dried material was concentrated under reduced pressure to give 43.1 g (quantitative) of 10-phthalyl-1,5-dibenzyloxycarbonyl-1,5,10-triazadecane as a pale yellow oily substance.

NMR ($CDCl_3$): δ = 1.3—2.1 (b, 6H), 1.9—3.9 (m, 8H), 5.10 (s, 4H), 7.30, 7.33 (s, s, 10H), 7.73 (m, 4H).

31.3 g (57.6 mmols) of the resulting 10-phthalyl-1,5-dibenzyloxycarbonyl-1,5,10-triazedecane was dissolved in 600 ml of ethanol. 18.2 g (291 mmols) of 80% hydrozine hydrate was added to the solution, and the mixture was heated overnight under reflux. Crystals that were precipitated were removed by filtration, and the filtrate was concentrated of under reduced pressure.

The residue was dissolved in 300 ml of ethyl acetate, and the solution was extracted with dilute hydrochloric acid to transfer the desired product into the aqueous layer. The aqueous layer was washed with ethyl acetate, and sodium carbonate was added to adjust the aqueous liquid to pH 10. An oily material that separated out was extracted with 500 ml of ethyl acetate, and the ethyl acetate layer was washed with a saturated aqueous solution of sodium chloride. The washed layer was dried over anhydrous sodium sulfate, and concentrated under reduced pressure to obtain 20.1 g of 1,5-dibenzyloxycarbonyl-1,5,10-triazadecane in a yield of 84.8%.

NMR ($CDCl_3$): δ = 1.0—2.3 (b, 8H), 2.3—2.9 (b, 2H), 2.9—3.5 (m, 6H), 5.05 (s, 2H), 5.07 (s, 2H), 5.1—6.1 (b, 1H), 7.30 (s, 10H).

(2) 10-Glycyl-1,5-dibenzyloxycarbonyl-1,5,10-triazedecane

370 ml of ethanol and 6.00 g (120 mmols) of hydrazine hydrate were added to 14.4 g (24.0 mmols) of 10-(N,N-phthalylglycyl)-1,5-dibenzyloxycarbonyl-1,5,10-triazedecane, and the mixture was refluxed for 2 hours. After the reaction, the insolubles were removed by filtration, and the filtrate was conc entrated under reduced pressure. The resulting oily material was dissolved in 300 ml of ethyl acetate, and the solution was

washed with a 5% aqueous solution of sodium hydrogencarbonate and distilled water in this order. The ethyl acetate layer was dried over anhydrous sodium sulfate, and the desiccant was removed by filtration. The filtrate was concentrated under reduced pressure to obtain 12.5 g of the desired product as an oily substance in a quantitative yield.

NMR ($CDCl_3$): δ = 0.8—2.1 (b, 6H), 2.8—3.5 (b, 10H), 5.0—6.1 (b, 2H), 5.06 (s, 2H), 5.10 (s, 2H), 7.33 (s, 10H).

TLC (chloroform:methanol:acetic acid = 95:5:3 v/v): Rf = 0.1.

(3) 10-[N-(7-guanidinoheptanoyl)glycyl]-1,5-dibenzyloxycarbonyl-1,5,10-triazedecane hydrochloride

12.5 g (about 24 mmols) of 10-glycyl-1,5-dibenzyloxycarbonyl-1,5,10-triazedecane was dissolved in 20 ml of tetrahydrofuran. 4.00 ml (29.0 mmols) of triethylamine was added to the solution under cooling with ice. To the mixture was further added a solution of 1.3 g (about 30 mmols) of an ester of 7-guanidinoheptanoic acid hydrochloride with N-hydroxysuccinimide dissolved in 50 ml of dimethylformamide, and the system was reacted overnight at room temperature.

The reaction mixture was concentrated under reduced pressure, and the oily residue was dissolved in 700 ml of ethyl acetate. The solution was washed with 0.5N hydrochloric acid saturated with sodium chloride, and then washed with a saturated aqueous solution of sodium chloride. An oily material that was precipitated during this washing step was dissolved by addition of a small amount of ethanol. Then, the ethyl acetate layer was dried over anhydrous sodium sulfate, and the desiccant was removed by filtration. The filtrate was concentrated under reduced pressure to obtain 20.8 g (yield: quantitative) of the desired product as an oily substance.

NMR ($CDCl_3$): = 0.8—1.9 (b, 14H), 1.9—2.4 (b, 2H), 2.7—3.5 (b, 10H), 3.5—4.0 (b, 2H), 5.04 (s, 4H), 6.2—8.0 (b, 8H), 7.27 (s, 10H).

TLC (n-butanol:acetic acid:water = 4:1:1 v/v): Rf = 0.3.

The ester of 7-guanidinoheptanoic acid hydrochloride with N-hydroxysuccinimide used above was synthesized in the following way:

50.0 g (0.344 mol) of 7-aminoheptanoic acid and 63.5 g (0.514 mol) of methylisourea were dissolved in 250 ml of 50% hydrous methanol (v/v. To the solution was added dropwise a solution of 34.3 g (8.858 mol) of sodium hydroxide in 400 ml of water.

After the dropwise addition, the mixture was heated overnight under reflux. The reaction mixture was concentrated under reduced pressure to about a half of the original quantity, followed by cooling the concentrate to precipitate white crystals. The crystals were collected by filtration, and dried to give 42.6 g (yield: 66.1%) of 7-guanidinoheptanoic acid.

NMR ($D_2O$+DCl): δ = 1.0—2.0 (b, 8H), 2.2—2.6 (m, 2H), 3.0—3.3 (m, 2H).

TLC (chloroform:methanol:17% ammonia water = 2:2:1): Rf = 0.5.

9-guanidinononanoic acid was synthesized likewise from 9-aminononanoic acid.

NMR (DMSO+$d_6$+DCl): δ = 0.9—1.9 (b, 12H), 2.0—2.4 (m, 2H), 2.9—3.4 (m, 2H).

TLC (n-propanol:water:29% ammonia water = 10:3:0.15 v/v): Rf = 0.6.

18.7 g (10 mmols) of 7-guanidinoheptanoic acid obtained in the preceding step was dissolved in 1N hydrochloric acid, and the solution was concentrated under reduced pressure to distil off water and bring about sufficient dryness, thereby obtaining 2.24 g (10 mmols) of 7-guanidinoheptanoic acid hydrochloride. This product was dissolved in 10 ml of dry dimethylformamide, and 2.06 g (10 mmols) of dicyclohexylcarbodiimide and 1.00 g (12 mmols) of N-hydroxysyccinimide were added in this order to the solution with stirring and cooling with ice. The reaction system was stirred for 30 minutes at 0°C, then returned to room temperature, and reacted overnight with stirring. Dicyclohexylurea that was precipitated was removed by filtration, and the filtrate was concentrated under reduced pressure.

10 ml of ethyl acetate to the residue, and dicyclohexylurea formed as a precipitate was removed by filtration, whereafter the filtrate was concentrated under reduced pressure. Petroleum ether was added to the resulting oily material, and the mixture was stirred, followed by decanting the supernatant. This washing step was repeated several times, and the washed oily material was concentracted under reduced pressure. The remaining solvent was removed by means of a vacuum pump to obtain 3.65 g (quantitative) of a crude ester of 7-guanidinoheptanoic acid hydrochloride with N-hydroxysuccinimide.

NMR (DMSO-$d_6$): δ = 1.1—2.0 (b, 8H), 2.67 (t, 2H, J=6.0Hz), 2.84 (s, 4H), 3.1 (m, 2H), 7.3, 8.0 (b, b, 5H).

## Referential Example 2

(1) 10-(N-tert-butoxycarbonyl-O-benzyl-L-seryl)-1,5-dibenzyloxycarbonyl-1,5,10-triazadecane

4.76 g (11.5 mmols) of 1,5-dibenzyloxycarbonyl-1,5,10-triazadecane was dissolved in 50 ml of ethyl acetate, and 1.04 g (10.3 mmols) of triethylamine was added to the solution under cooling with ice. 5.87 g (about 15 mmols) of an ester of N-tert-butoxycarbonyl-O-benzyl-L-serine with N-hydroxysuccinimide was further added to the mixture, and the reaction system was reacted overnight at room temperature. 50 ml of ethyl acetate was added to the reaction mixture, and the resulting ethyl acetate solution was washed with a 5% aqueous solution of sodium hydrogencarbonate, 0.1N hydrochloric acid, and a saturated aqueous solution of sodium chloride in this order. The ethyl acetate layer was dried over anhydrous sodium sulfate,

and the desiccant was removed by filtration. Concentrating the filtrate under reduced pressure gave 8.34 g (yield: quantitative) of the desired product.

NMR ($CDCl_3$): δ = 8.8—2.2 (b, 6H), 1.46 (s, 9H), 2.7—3.5 (b, 8H), 3.66 (m, 2H), 3.9—4.4 (b, 1H), 4.50 (s, 2H), 5.11 (s, 4H), 5.1—5.4 (b, 2H), 6.1—6.8 (b, 1H), 7.33 (s, 15H).

TLC (chloroform:methanol = 9:1 v/v): Rf = 0.8.

(2) 10-(O-benzyl-L-seryl)-1,5-dibenzyloxycarbonyl-1,5,10-triazedecane

8.00 g (11.5 mmols) of 10-(N-tert-butoxycarbonyl-O-benzyl-L-seryl)-1,5-dibenzyloxycarbonyl-1,5,10-triazdecane was dissolved by the addition of 8.0 ml of trifluoroacetic acid, and the system was reacted for 3 hours at room temperature. The reaction mixture was concentrated under reduced pressure, and the residual oil was dissolved in 200 ml of ethyl acetate. The solution was washed with a 5% solution of sodium hydrogencarbonate and distilled water in this order, and the ethyl acetate layer was dried over anhydrous sodium sulfate. After the desiccant was removed by filtration, the filtrate was concentrated under reduced pressure to obtain 6.82 g (yield: quantitative) of the desired product as an oily substance.

NMR ($CDCl_3$): δ = 1.2—2.0 (b, 6H), 1.74 (s, 2H), 2.8—3.5 (b, 8H), 3.64 (m, 3H), 4.51 (s, 2H), 5.12 (s, 4H), 4.6—6.0 (b, 2H), 7.33 (s, 15H).

TLC (chloroform:methanol = 9:1 v/v): Rf = 0.5.

(3) 10-[N-(7-guanidinoheptanoyl)-O-benzyl-L-seryl]-1,5-dibenzyloxycarbonyl-1,5,10-triazedecane

3.56 g (6.03 mmols) of 10-(O-benzyl-L-seryl-1,5-dibenzyloxycarbonyl-1,5,10-triazadecane was dissolved in 30 ml of tetrahydrofuran. With the solution cooled with ice, 0.61 g (6.03 mmols) of triethylamine was added. To the mixture was further added a solution of 6.14 g (about 7 mmols) of a trifluoroacetate of an ester of 7-guanidinoheptanoic acid with p-nitrophenol dissolved in 10 ml of tetrahydrofuran.

This was reacted overnight at room temperature. The reaction mixture was concentrated under reduced pressure, and the residual oil was dissolved in 150 ml of ethyl acetate. The solution was washed with a 10% aqueous solution of sodium carbonate, 0.5N hydrochloric acid, and a saturated aqueous solution of sodium chloride in this order. During the washing step, oily matter was formed as a precipitate, which was dissolved by addition of a small amount of ethanol. Then, the ethyl acetate layer was dried over anhydrous sodium sulfate, and the desiccant was removed by filtration. Concentrating the filtrate under reduced pressure gave 4.77 g (yield: quantitative) of the desired product as an oily substance.

NMR (DMSO-$d_6$): δ = 1.0—2.0 (b, 14H), 2.0—2.4 (b, 2H), 2.7—3.5 (b, 10H), 3.58 (d, 2H, J=5Hz), 4.2—4.8 (b, 1H), 4.47 (s, 2H), 5.01 (s, 2H), 5.04 (s, 2H), 6.8—8.3 (b, 8H), 7.27 (s, 5H), 7.30 (s, 10H).

TLC (chloroform:methanol:acetic acid = 9:1:0.3 v/v): Rf = 0.2.

The procedure of Referential Example 1 or 2 was performed using as N-protected amino acids of general formula (IV) N-tert-butoxycarbonyl-O-benzyl-D-serine, N-tert-butoxycarbonyl-L-alanine, N-tert-butoxycarbonyl-β-alanine, N-tert-butoxycarbonyl-γ-aminobutyric acid, N-tert-butoxycarbonyl-L-proline, N-tert-butoxycarbonyl-L-leucine, N-tert-butoxycarbonyl-L-aspartic acid-β-benzyl ester, N-tert-butoxycarbonyl-L-glutamide, N-tert-butoxycarbonyl-$N^G$-nitro-L-arginine, N-tert-butoxycarbonyl-L-phenylalanine, N-butoxycarbonyl-$N^{im}$-benzyloxycarbonyl-L-histidine, and N-tert-butoxycarbonyl O-benzyl-L-threonine. The corresponding 10-[N-(7-guanidinoheptanoyl)-$R_2'$]-1,5-dibenzyloxycarbonyl-1,5,10-triazadecane hydrochloride of the general formula (II) were obtained.

Referential Example 3

(S)-7-guanidine-3-acetyloxyheptanoic acid

3.55 g (14.8 mmols) of (S)-7-guanidino-3-acetyloxyheptanoic acid was dissolved in 100 ml of glacial acetic acid, and hydrogen chloride gas was introduced to saturation at a temperature lower than room temperature. The reaction mixture was stirred for 2 hours at room temperature, and concentrated under reduced pressure. The above procedure was carried out two more times, and the residue was sufficiently dried to obtain the desired product in a quantitative amount.

NMR (DMSO-$d_6$): δ = 1.0—1.9 (b, 6H), 2.00 (s, 3H), 2.4—2.8 (b, 2H), 2.9—3.4 (b, 2H), 4.9—5.3 (b, 1H), 6.6—9.3 (b, 6H).

TLC (alumina plate:n-butanol:pyridine:water:acetic acid = 6:4:3:1 v/v): Rf = 0.5.

Referential Example 4

(1) 10-(N-benzyloxycarbonyl-DL-homoseryl)-1,5-di-tert-butoxycarbonyl-1,5,10-triazedecane

3.76 g (16.0 mmols) of N-benzyloxycarbonyl-DL-homoserine lactone was dissolved in 20 ml of tetrahydrofuran. To the resulting solution was added a solution of 2.07 g (6.00 mmols) of 1,5-di-tert-butoxycarbonyl-1,5,10-triazedecane in 5 ml of tetrahydrofuran. The system was reacted for 3 days at room temperature.

The reaction mixture was concentrated under reduced pressure to evaporate the solvent. The residual oil was column-chromatographed on 400 g of silica gel (Wako-Gel® C—200) using a mixture of chloroform and methanol [chloroform:methanol = 20:1 v/v)] as a developer. Active fractions were concentrated under reduced pressure to obtain 1.38 g (yield: 37.8%) of the desired product.

NMR ($CDCl_3$): δ = 1.0—2.3 (b, 8H), 1.47 (s, 18H), 2.8—3.5 (b, 8H), 3.5—4.0 (b, 3H), 4.1—4.6 (m, 1H), 4.7—5.4 (b, 1H), 5.09 (s, 2H), 5.8—6.3 (b, 1H), 6.6—7.1 (b, 1H), 7.31 (s, 5H).

TLC (chloroform:methanol = 20:1 v/v): Rf = 0.2.

(2) 10-(DL-homoseryl)-1,5-di-tert-butoxycarbonyl-1,5,10-triazadecane

1.35 g (2.22 mmols) of 10-(N-benzyloxycarbonyl-DL-homoseryl)-1,5,-di-tert-butoxycarbonyl-1,5,10-triazedecane was dissolved in 20 ml of methanol. 0.1 g of palladium black was added to the solution, and the system was catalytically reduced for 8 hours at room temperature and atmospheric pressure. After the reaction, the catalyst was removed by filtration, and the filtrate was concentrated under reduced pressure to distill off the solvent. 0.91 g (yield: 86.4%) of the desired product was obtained as the residual oil.

NMR ($CDCl_3$): δ = 1.1—2.2 (b, 10H), 1.47 (s, 18H), 2.8—3.5 (b, 9H), 3.5—4.0 (m, 3H), 4.6—5.6 (b, 1H), 7.4—7.9 (b, 1H).

TLC (chloroform:methanol = 9:1 v/v): Rf = 0.1.

(3) 10-[N-(7-guanidinoheptanoyl)-DL-homoseryl]-1,5-di-tert-butoxycarbonyl-1,5,10-triazadecane acetate

0.88 g (1.85 mmols) of 10-(DL-homoseryl)-1,5-di-tert-butoxycarbonyl-1,5,10-triazadecane was dissolved in 5 ml of tetrahydrofuran, and 0.30 g (2.96 mmols) of triethylamine was added to the solution under cooling with ice. A solution of 1.19 g (about 3 mmols) of an ester of 7-guanidino-heptanoic acid hydrochloride with N-hydroxysuccinimide dissolved in 8 ml of dimethylformamide was further added to the mixture, and the system was reacted overnight at room temperature.

After the reaction, the reaction mixture was concentrated under reduced pressure. The residual oil was dissolved in 50 ml of a 2% aqueous solution of phosphoric acid, and the solution was washed with ethyl acetate. Sodium carbonate was added to the aqueous layer to adjust it to about pH 10.5. Then, the aqueous layer was extracted twice with 50 ml of ethyl acetate, and acetic acid was added to the ethyl acetate layer until it became susbtantially neutral. Concentrating the mixture under reduced pressure gave 1.08 g (yield: 86.4%) of the desired product as an oily substance.

NMR (DMSO-$d_6$): δ = 0.9—2.0 (b, 16H), 1.43 (s, 18H), 1.84 (s, 3H), 2.0—2.4 (b, 2H), 2.7—3.7 (b, 11H), 3.37 (t, 2H), 4.1—4.6 (b, 1H), 6.3—8.4 (b, 8H).

TLC (chloroform:methanol:acetic acid = 8:2:0.5 v/v): Rf = 0.3.

Referential Example 5

10-[N-(S)-7-guanidino-3-acetoxyheptanoyl)glycyl]-1,5-dibenzyloxycarbonyl-1,5,10-triazadecane hydrochloride

A solution of 1.28 g (3.1 mmols) of 1,5-dibenzyloxycarbonyl-1,5,10-triazadecane dissolved in 20 ml of tetrahydrofuran and 0.5 g of triethylamine were added to a solution of an ester (3.1 mmols) of N-(7-guanidino-3-acetoxyheptanoyl)glycine hydrochloride with N-hydroxysuccinimide dissolved in 50 ml of dimethylformamide. The system was reacted overnight at room temperature. The reaction mixture was concentrated under reduced pressure. The residual oil was dissolved in 100 ml of ethyl acetate. The solution was washed with 0.5N hydrochloric acid and a saturated aqueous solution of sodium chloride in this order, with ethanol added little by little to avoid the precipitation of oily matter. The ethyl acetate layer was dried over anhydrous magnesium sulfate, and the desiccant was removed by filtration. The filtrate was concentrated under reduced pressure to obtain 1.91 g (yield: 83.9%) of the desired product as an oily substance.

Referential Example 6

10-[N-(9-guanidinononanoyl)-O-benzyl-L-seryl)-1,5-benzyloxycarbonyl-1,5,10-triazadecane hydrochloride

4.71 g (7.98 mmols) of 10-(O-benzyl-L-seryl)-1,5-benzyloxycarbonyl-1,5,10-triazadecane hydrochloride was dissolved in 30 ml of dimethylformamide, and 1.30 g (12.8 mmols) of triethylamine was added to the solution under cooling with ice. A dimethylformamide solution containing an ester (about 12 mmols) of 9-guanidinononanoic acid hydrochloride with N-hydroxysuccinimide was further added. The resulting system was reacted overnight. The reaction mixture was concentrated under reduced pressure, and the oily residue was dissolved in 500 ml of ethyl acetate. The solution was washed with a 5% solution of sodium hydrogencarbonate, a saturated aqueous solution of sodium chloride, 0.2N hydrochloric acid, and a saturated aqueous solution of sodium chloride in this order, with ethanol being added little by little to avoid the precipitation of oily matter. The ethyl acetate layer was concentrated under reduced pressure to obtain 6.02 g (yield: 95.7%) of the desired product as an oily substance.

**Claims for the Contracting States: BE CH DE FR GB IT LI LU NL SE**

1. A spergualin-related compound of the following general formula I

$$H_2N\underset{\underset{NH}{\parallel}}{C}NH(CH_2)_m\underset{\underset{R_1}{|}}{C}HCH_2CO{-}R_2{-}NH(CH_2)_4NH(CH_2)_3NH_2 \qquad (I)$$

wherein $R_1$ represents a hydrogen atom, a hydroxyl group, or an aliphatic acyloxy group with 1 to 10 carbon atoms; $R_2$ represents a residue of an α- or ω-amino acid remaining after removing one of the hydrogen atoms from the α- or ω-amino group and the hydroxyl group from the α-carboxyl group, having the formula IV

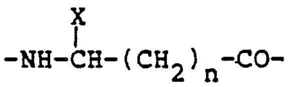

(IV)

where X represents a hydrogen atom or a straight-chain or branched alkyl group having 1 to 6 carbon atoms, said alkyl group optionally having as a substituent a hydroxyl, carboxyl, lower alkoxycarbonyl, guanidino, phenyl or imidazole group and n denotes an integer of 0 to 2; or having the formula V

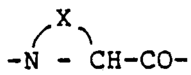

(V)

wherein X represents an alkylene group having up to 3 carbon atoms; said $R_2$ being attached to the adjacent carbonyl group and amino group by amide bonds; and m denotes an integer of 4 to 6, and a physiologically acceptable salt thereof.

2. A compound as claimed in claim 1 wherein $R_2$ is a residue of the amino acids glycine, alanine, α-aminobutyric acid, proline, leucine, serine, homoserine, threonine, aspartic acid, glutamic acid, arginine, phenylalanine, histidine or β-alanine.

3. A process for preparing a compound of the formula I as defined in claims 1 or 2, which comprises removing protective groups from a protected compound of the formula

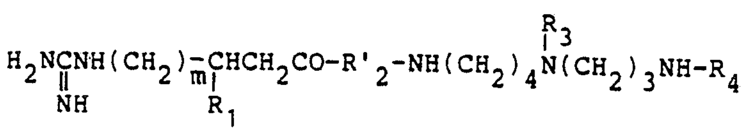

(II)

wherein $R_1$ is as defined; $R_2'$ has the meaning of $R_2$ with the proviso, that said α- or ω-amino acid is optionally protected, if it has a functional group as a substituent; $R_3$ and $R_4$ are identical or different and each represents a protective group for the amino group, and m is as defined, by usual methods.

4. A pharmaceutical composition which comprises as active-ingredient a compound of the formula (I) as claimed in claim 1 or a physiologically acceptable salt thereof in association with a pharmaceutically acceptable carrier and/or auxiliary.

5. Use of a compound as claimed in claim 1 for the preparation of anti-tumor agents having high stability.

**Claim for the Contracting State: AT**

A process for preparing a spergualin-related compound of the following general formula I

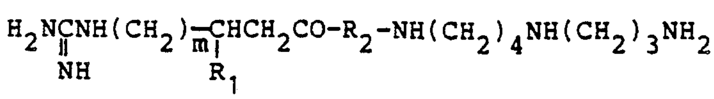

(I)

wherein $R_1$ represents a hydrogen atom, a hydroxyl group, or an aliphatic acyloxy group with 1 to 10 carbon atoms; $R_2$ represents a residue of an α- or ω-amino acid remaining after removing one of the hydrogen atoms from the α- or ω-amino group and the hydroxyl group from the α-carboxyl group, having the formula IV

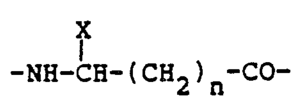

(IV)

where X represents a hydrogen atom or a straight-chain or branched alkyl group having 1 to 6 carbon atoms, said alkyl group optionally having as a substituent a hydroxyl, carboxyl, lower alkoxycarbonyl, guanidino, phenyl or imidazole group and n denotes an integer of 0 to 2; or having the formula V

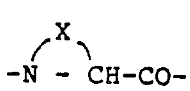

(V)

wherein X represents an alkylene group having up to 3 carbon atoms; said $R_2$ being attached to the adjacent carbonyl group and amino group by amide bonds; and m denotes an integer of 4 to 6, and a

physiologically acceptable salt thereof, which comprises removing protective groups from a protected compound of the formula

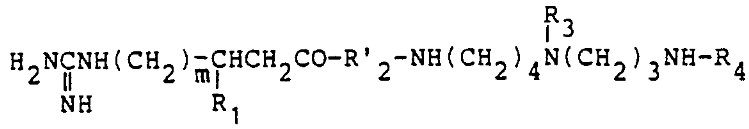

(II

wherein $R_1$ is as defined; $R_2'$ has the meaning of $R_2$ with the proviso, that said α- or ω-amino acid is optionally protected, if it has a functional group as a substituent; $R_3$ and $R_4$ are identical or different and each represents a protective group for the amino group, and m is as defined, by usual methods.

**Patentansprüche für die Vertragsstaaten: BE CH DE FR GB LI LU NL SE**

1. Spergualinähnliche Verbindungen der folgenden allgemeinen Formel I

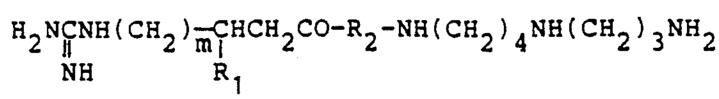

(I)

worin $R_1$ ein Wasserstoffatom, eine Hydroxylgruppe oder eine aliphatische Acyloxygruppe mit 1 bis 10 C-Atomen darstellt, $R_2$ einen, nach Abspaltung eines der Wasserstoffatome von der α- oder ω-Aminogruppe sowie der Hydroxylgruppe von der α-Carboxylgruppe verbleibenden Rest einer α- oder ω-Aminosäure mit der Formel IV

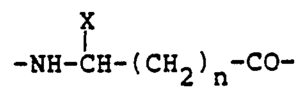

(IV)

wo X ein Wasserstoffatom oder eine geradkettige oder verzweigte Alkylgruppe mit 1 bis 6 C-Atomen darstellt, wobei besagte Alkylgruppe gegebenenfalls eine Hydroxyl-, Carboxyl-, niedere Alkoxycarbonyl-, Guanidiono-, Phenyl- oder Imidazolgruppe als einen Substituuenten aufweist und n eine ganze Zahl von 0 bis 2 bedeutet, oder mit der Formel V

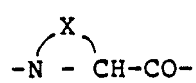

(V)

worin X eine Alkylengruppe mit bis zu 3 C-Atomen darstellt, besagter $R_2$ mit der benachbarten Carbonyl- und Aminogruppe über Amidbindungen verbunden ist und m eine ganze Zahl von 4 bis 6 bedeutet, und eines ihrer physiologisch unbedenklichen Salze.

2. Verbindungen nach Anspruch 1, worin $R_2$ ein Rest der Aminosäuren Glycin, Alanin, α-Aminobuttersäure, Prolin, Leucin, Serin, Homoserin, Threonin, Asparaginsäure, Glutaminsäure, Arginin, Phenylalanin, Histidin oder β-Alanin bedeutet.

3. Verfahren zur Herstellung einer Verbindung der Formel I nach Anspruch 1 oder 2, dadurch gekennzeichnet, daß man Schutzgruppen von einer geschützten Verbindung der Formel

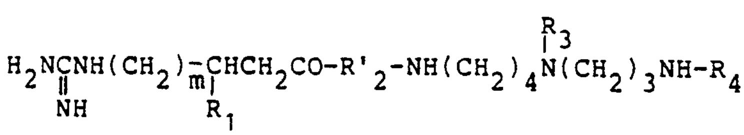

(II)

worin $R_1$ die genannte Bedeutung hat, $R_2$ die Bedeutung von $R_2$ hat mit dem Vorbehalt, daß falls basagte α- oder ω-Aminosäure eine funktionelle Gruppe als einen Substituenten hat, diese gegebenenfalls geschützt ist, $R_3$ und $R_4$ identisch oder verschieden sind und jeweils eine Schutzgruppe für die Aminogruppe darstellen und m die genannte Bedeutung hat, mit üblichen Methoden abspaltet.

4. Pharmazeutische Zusammensetzungen, welche als aktiven Bestandteil eine Verbindung der Formel (I) nach Anpruch 1 oder ein physiologisch unbedenkliches Salz davon in Verbindung mit einem physiologisch unbedenklichen Träger- und/oder Hilfmittel enthalten.

5. Verwendung einer Verbindung nach Anspruch 1 zur Zubereitung eines Antitumormittels mit hoher Stabilität.

**Patentanspruch für den Vertragsstaat: AT**

Verfahren zur Herstellung einer sperguaIinähnlichen Verbindung der folgenden allgemeinen Formel I

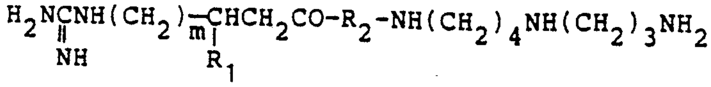

(I)

worin $R_1$ ein Wasserstoffatom, eine Hydroxylgruppe oder eine aliphatische Acyloxygruppe mit 1 bis 10 C-Atomen darstellt, $R_2$ einen, nach Abspaltung eines der Wasserstoffatome von der α- oder ω-Aminogruppe sowie der Hydroxylgruppe von der α-Carboxylgruppe verbleibenden Rest einer α- oder ω-Aminosäure mit der Formel IV

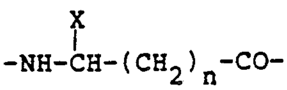

(IV)

wo X ein Wasserstoffatom oder eine geradkettige oder verzweigte Alkylgruppe mit 1 bis 6 C-Atomen darstellt, wobei besagte Alkylgruppe gegebenenfalls eine Hydroxyl-, Carboxyl-, niedere Alkoxycarbonyl-, Guanidiono-, Phenyl- oder Imidazolgruppe als einen Substituenten aufweist und n eine ganze Zahl von 0 bis 2 bedeutet, oder mit der Formel V

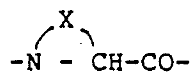

(V)

worin X eine Alkylengruppe mit bis zu 3 C-Atomen darstellt, besagter $R_2$ mit der benachbarten Carbonyl- und Aminogruppe über Amidbindungen verbunden ist und m eine ganze Zahl von 4 bis 6 bedeutet, und eines ihrer physiologisch unbedenklichen Salze, dadurch gekennzeichnet, daß man Schutzgruppen von einer geschützten Verbindung der Formel

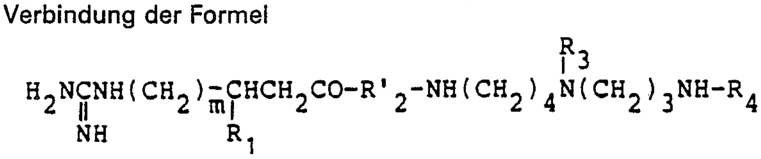

(II)

worin $R_1$ die genannte Bedeutung hat, $R_2$ die Bedeutung von $R_2$ hat mit dem Vorbehalt, daß falls basagte α- oder ω-Aminosäure eine funktionelle Gruppe als einen Substituenten hat, diese gegebenenfalls geschützt ist, $R_3$ und $R_4$ identisch oder verschieden sind und jeweils eine Schutzgruppe für die Aminogruppe darstellen und m die genannte Bedeutung hat, mit üblichen Methoden abspaltet.

**Revendications pour les Etats contractants: BE CH DE FR GB LI LU NL SE**

1. Composé apparenté à la spergualine, de formule générale I

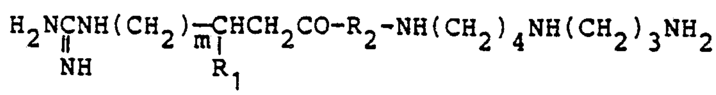

(I)

dans laquelle $R_1$ représente un atome d'hydrogène, un groupe hydroxy ou un groupe acyloxy aliphatique ayant de 1 à 10 atomes de carbone; $R_2$ représente le reste d'un acide α- ou ω-aminé, restant après l'élimination d'un des atomes d'hydrogène du groupe α- ou ω-amino et du groupe hydroxy du groupe α-carboxy, correspondant à la formule IV

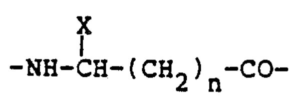

(IV)

dans laquelle X représente un atome d'hydrogène ou un groupe alkyle à chaîne droite ou ramifiée ayant de 1 à 6 atomes de carbone, ledit groupe alkyle comportant éventuellement en tant que substituant un groupe hydroxy, carboxy, alcoxy-(inférieur)-carbonyle, guanidino, phényle ou imidazole; et n représente un nombre entier 0 à 2; ou correspondant à la formule V

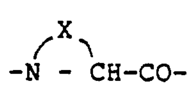

(V)

dans laquelle X représente un groupe alkyklène ayant jusqu'à 3 atomes de carbone; ledit radical $R_2$ étant lié au groupe carbonyle et un groupe amino adjacents par des liaisons amide; et m représente un nombre entier de 4 à 6; et sel physiologiquement acceptable de celui-ci.

2. Composé selon la revendication 1, dans lequel $R_2$ est un reste des acides aminés glycine, alanine, acide 33-aminobutyrique, proline, leucine, sérine, homo sérine, thréonine, acide aspartique, acide glutamique, arginine, phénylalanine, histidine ou β-alanine;

3. Procédé pour la préparation d'un composé de formule I, tel que défini dans la revendication 1 ou 2, lequel comprend l'elimination, par des procédés usuels, de groupements protecteurs hors d'un composé de formule

$$H_2N\underset{\underset{NH}{\|}}{C}NH(CH_2)_m\underset{\underset{R_1}{|}}{C}HCH_2CO-R'_2-NH(CH_2)_4\overset{\overset{R_3}{|}}{N}(CH_2)_3NH-R_4 \qquad (II)$$

dans laquelle $R_1$ est tel que défini plus haut; $R_2'$ a la signification de $R_2$, étant entendu que ledit acide α- ou ω-aminé est éventuellement protégé s'il comporte un groupe fonctionnel en tant que substituant; $R_3$ et $R_4$ sont identiques ou différents, et représentent chacun un groupement protecteur pour le groupe amino; et m est tel que défini plus haut.

4. Composition pharmaceutique comprenant en tant que composant actif un composé de formule (I) selon la revendication 1 ou un sel physiologiquement acceptable de celui-ci, en association avec un véhicule et/ou adjuvant pharmaceutiquement acceptable.

5. Utilisation d'un composé selon la revendication 1, pour la préparation d'agents antitumoraux ayant une haute stabilité.

**Revendication pour l'Etat contractant: AT**

Procédé pour la préparation d'un composé apparenté à la spergualine, de formule générale I

$$H_2N\underset{\underset{NH}{\|}}{C}NH(CH_2)_m\underset{\underset{R_1}{|}}{C}HCH_2CO-R_2-NH(CH_2)_4NH(CH_2)_3NH_2 \qquad (I)$$

dans laquelle $R_1$ représente un atome d'hydrogène, un groupe hydroxy ou un groupe acyloxy aliphatique ayant de 1 à 10 atomes de carbone; $R_2$ représente le reste d'un acide α- ou ω-aminé, restant après l'élimination d'un des atomes d'hydrogène du groupe α- ou ω-amino et du groupe hydroxy du groupe α-carboxy, correspondant à la formule IV

$$-NH-\overset{\overset{X}{|}}{C}H-(CH_2)_n-CO- \qquad (IV)$$

dans laquelle X représente un atome d'hydrogène ou un groupe alkyle à chaîne droite ou ramifiée ayant de 1 à 6 atomes de carbone, ledit groupe alkyle comportant éventuellement en tant que substituant un groupe hydroxy, carboxy, alcoxy-(inférieur)-carbonyle, guanidino, phényle ou imidazole; et n représente un nombre entier 0 à 2; ou correspondant à la formule V

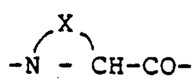

(V)

dans laquelle X représente un groupe alkyklène ayant jusqu'à 3 atomes de carbone; ledit radical $R_2$ étant lié au groupe carbonyle et un groupe amino adjacents par des liaisons amide; et m représente un nombre entier de 4 à 6; et sel physiologiquement acceptable de celui-ci, lequel comprend l'elimination, par des procédés usuels de groupements protecteurs hors d'un composé protégé de formule

$$H_2N\underset{\underset{NH}{\|}}{C}NH(CH_2)_m\underset{\underset{R_1}{|}}{C}HCH_2CO-R'_2-NH(CH_2)_4\overset{\overset{R_3}{|}}{N}(CH_2)_3NH-R_4 \qquad (II)$$

dans laquelle $R_1$ est tel que défini plus haut; $R_2'$ a la signification de $R_2$, étant entendu que ledit acide α- ou ω-aminé est éventuellement protégé s'il comporte un groupe fonctionnel en tant que substituant; $R_3$ et $R_4$ sont identiques ou différents, et représentent chacun un groupement protecteur pour le groupe amino; et m est tel que défini plus haut.